# EUROPEAN PATENT APPLICATION

(11) **EP 1 876 183 A1**
(43) Date of publication of application: **09.01.2008**
(21) Application number: 06013852.6
(22) Date of filing: 04.07.2006
(51) Int. Cl.: C07K 7/06, C07K 14/755, A61K 38/08, G01N 33/68

(54) **Minimized small peptides with high affinity for factor VIII and factor VIII-like proteins**

(71) Applicant: Technische Universität München, 80333 München (DE)
(72) Inventor: Knör, Sebastian, 80687 München (DE); Kessler, Horst, 85748 Garching (DE); Hauser, Charlotte, Dr., 80639 München (DE); Saenko, Evgueni, 119454 Moscow (RU); Khrenov, Alexey, North Potomac Maryland 20878 (US)
(74) Representative: HOFFMANN EITLE

(57) **Abstract**

The present invention relates to the composition of small molecules and their use in the field of protein isolation, purification, stabilizing and/or enhancing its activity. In particular, the present invention relates to the synthesis and optimization of compounds comprising small peptides and peptide derivatives with affinity to coagulation Factor VIII and/or Factor VIII-like polypeptides and/or domains thereof. These compounds are useful for labeling, detecting, identifying, isolating and preferably for purifying, stabilizing and enhancing the activity of Factor VIII, Factor VIII-like polypeptides or domains thereof from physiological and non-physiological solutions comprising same. Further, these compounds may be used as ligands, which bind Factor VIII, Factor VIII-like polypeptides or domains thereof in methods of the present invention.

## Description

### FIELD OF THE INVENTION

The present invention is related to the composition of small molecules and their use in the field of protein isolation, purification, stabilizing and/or enhancing its activity.

In particular, the present invention relates to the synthesis and optimization of compounds comprising small peptides and peptide derivatives with affinity to coagulation Factor VIII and/or Factor VIII-like polypeptides and/or domains thereof. These compounds are useful for labeling, detecting, identifying, isolating and preferably for purifying, stabilizing and enhancing the activity of Factor VIII, Factor VIII-like polypeptides or domains thereof from physiological and non-physiological solutions comprising same. Further, these compounds may be used as ligands, which bind Factor VIII, Factor VIII-like polypeptides or domains thereof in methods of the present invention.

For the purpose of the present invention all references as cited herein are incorporated by reference in their entireties.

### BACKGROUND

Factor VIII (FVIII) is an essential component of the intrinsic pathway of blood coagulation (Bolton-Maggs, P. H. B.; K. J. Pasi Lancet 2003, 361, 1801). This plasma protein is circulating in blood in complex with von Willebrand factor (vWf), which protects and stabilizes it. Genetic deficiency of FVIII function results in a life-threatening bleeding disorder known as Hemophilia A, one of the most common bleeding disorders, which is treated by repeated infusions of FVIII. Hemophilia A is the result of an inherited deficiency of Factor VIII. For medical treatment, patients are given doses of Factor VIII derived from either blood plasma or recombinant cells.

Hemophilia A, the hereditary X chromosome-linked bleeding disorder caused by deficiency or structural defects in a coagulation Factor VIII (FVIII), affects approximately one in 5000 males. The clinical severity of Hemophilia A correlates with the degree of factor deficiency and is classified as severe disease with FVIII levels of less than 1%, moderate (1-5% FVIII levels) and mild disease ( 5-25% FVIII levels). The disease is characterized by spontaneous bleedings, as well as by uncontrollable bleedings in case of trauma or surgery. Other clinical hallmarks of Hemophilia A are acute recurrent painful hemarthrosis, which can progress to chronic arthropathy characterized by progressive destruction of the cartilage and the adjacent bone, muscle hematoma, intracerebral hemorrhages and hematuria (Klinge, J.; Ananyeva, N. M.; Hauser, C. A.; Saenko, E. L. Semin. Thromb. Hemost. 2002, 28, 309-322).

Hemophilia A is treated by repeated infusions of FVIII, derived from either human blood plasma or recombinant cells, expressing FVIII.

The FVIII molecule **(∼300** kDa, 2332 amino acid residues) consists of three homologous A domains, two homologous C domains and the unique B domain, which are arranged in the order of A1-A2-B-A3-C1-C2. Prior to its secretion into plasma, FVIII is processed intracellularly to a Me²⁺-linked heterodimer produced by cleavage at the B-A3 junction. This cleavage generates the heavy chain (HCh) consisting of the A1 (1-372), A2 (373-740) and B domains (741-1648) and the light chain (LCh) composed of the A3 (1690-2019), C1 (2020-2172) and C2 (2173-2332) domains. The resulting protein is heterologous in size due to a number of additional cleavages within the B domain, giving the molecules with B-domains of different length. The C-terminal portions of the A1 (amino acids 337-372) and A2 (amino acids 711-740) domains and the N-terminal portion of LCh (amino acids 1649-1689) contain a high number of negatively charged residues and are called acidic regions (AR1, AR2 and AR3, respectively).

In order to cope with existing demands for better supply of FVIII as well as to reduce the risk of viral and prionic contamination, the use of recombinant FVIII has been drastically increased in the past years (Ananyeva N., Khrenov A., Darr F., Summers R., Sarafanov A., Saenko E. Expert Opin.Pharmacother. 2004; 5:1061-1070). Since the isolation of the Factor VIII gene in 1984 (Vehar, G. A.; Keyt, B.; Eaton, D.; Rodriguez, H.; O'Brien, D. P.; Rotblat, F.; Oppermann, H.; Keck, R.; Wood, W. I.; Harkins, R. N. Nature 1984, 312, 337-342; Toole, J. J.; Knopf, J. L.; Wozney, J. M.; Sultzman, L. A.; Buecker, J. L.; Pittman, D. D.; Kaufman, R. J.; Brown, E.; Shoemaker, C.; Orr, E. C. Nature 1984, 312, 342-347), preparations of novel recombinant Factor VIII molecules have greatly improved. Moreover, deeper insights in structure-function relationship of Factor VIII as well as more sophisticated techniques in molecular biology have opened up new possibilities in the generation of recombinant Factor VIII.

Several therapeutic recombinant FVIII products are currently available as lyophilized concentrates.
(1) For the synthesis of RECOMBINATE (Baxter) and BIOCLATE (Centeon), the genes for FVIII and vWf have been inserted into Chinese hamster ovary cells (CHO). The vWf acts as stabilizer for FVIII in cell culture. The recombinant protein is purified by single immunoaffinity chromatography using a murine monoclonal antibody followed by two ion exchange chromatography steps to complete the purification process. The purified recombinant FVIII is finally stabilized by the addition of pasteurized human albumin. The purification process does not include a separate virus inactivation step (Kaufman, R. J.; Wasley, L. C.; Furie, B. C.; Furie, B.; Shoemaker, C. B. J. Biol. Chem. 1986, 261, 9622-9628).
(2) In the case of KOGENATE (Bayer) and HELIXATE (Centeon), the gene for Factor VIII has been inserted into an established cell line from baby hamster kidney (BHK). The secreted recombinant FVIII is processed by multiple purification steps, including two ion-exchange chromatography gel filtration and size exclusion chromatography, as well as double immunoaffinity chromatography using a murine monoclonal antibody. The purified protein is then stabilized by pasteurized human albumin. Virus inactivation is achieved by heat-treatment (Addiego, J. E. Jr.; Gomperts, E.; Liu, S. L.; Bailey, P.; Courter, S. G.; Lee, M. L.; Neslund, G. G.; Kingdon, H. S.; Griffith, M. J. Thrombosis and haemostasis 1992, 67, 19-27).
(3) KOGENATE FS (Bayer) has been developed as a second generation product. Different to KOGENATE, KOGENATE FS is cultured in cell culture medium containing recombinant insulin and Human Plasma Protein Solution (HPPS), but no proteins derived from animal sources.
(4) REFACTO (Wyeth-Ayerst Pharmacia and Upjohn) is the first licensed B domain deleted recombinant FVIII molecule (BDDrFVIII). The r-FVIII SQ gene which encodes a single chain 170 kDa polypeptide, was derived from full-length cDNA by removing the major part of the region encoding the B-domain. The r-FVIII SQ vector system was inserted into CHO cells and cultured in a serum-free medium supplemented with human albumin and recombinant insulin. The purification comprises five different chromatography steps including immunoaffinity with monoclonal antibodies directed to the heavy chain of FVIII, and a chemical solvent/detergent virus inactivation step (Eriksson, R. K.; Fenge, C.; Lindner-Olsson, E.; Ljungqvist, C.; Rosenquist, J.; Smeds, A. L.; Ostlin, A.; Charlebois, T.; Leonard, M.; Kelley, B. D.; Ljungqvist, A. Sem. Hematol. 2001, 38, 24-31).

It is important in the development of FVIII products to avoid any use of animal or human proteins in order to improve safety. In contrast to currently licensed recombinant FVIII preparations, next generation FVIII products will adapt production methods to culture media that do not contain any components of human or animal origin. Thus, the ultimate goal is an improvement in the production of FVIII to the point of completely avoiding any contact with components derived from animal or human raw materials. There is also a demand to improve purification methods for FVIII. Methods offering FVIII of high purity and activity obtained directly from various solutions such as blood or cell culture supernatants remain in demand, thereby, reducing the number of purification steps, and cost involved. New methods to gain FVIII in a faster, more efficient and cost-effective way remain unrealized by the current art.

Factor VIII is usually concentrated by affinity chromatography, employing monoclonal antibodies as ligands (Amatschek, K; Necina, R.; Hahn, R.; Schallaun, E.; Schwinn, H.; Josic, D.; Jungbauer, A. J. High Resol. Chromatogr. 2000, 23, 47-58). According to recent statements of the Medical and Advisory Council of the US National Hemophilia Foundation and of the World Federation of Hemophilia, all efforts should be made to eliminate human and bovine proteins from the manufacturing process of recombinant products (Medical and Scientific Advisory Council (MASAC) document #151 «MASAC RECOMMENDATIONS CONCERNING THE TREATMENT OF HEMOPHILIA AND OTHER BLEEDING DISORDERS», available at National Hemophilia Foundation website).

Use of oligo- and polypeptides as the partners of affinity ligands polypeptides has been suggested (see, WO 99/14232; or US 2003-165822, each incorporated herein by reference in their entirety). Nevertheless, this method still has disadvantages. First, the large scale synthesis and purification of the mentioned oligopeptides is not trivial and quiet cost-intensive. Furthermore, these oligopeptides are sensitive towards proteolytic degradation and the presence of proteases cannot be completely avoided if raw materials derived from blood or cell cultures are applied to the affinity column. This may rapidly lead to inefficiency and reduced selectivity of the affinity purification step and, furthermore, to a reduced purity and half life of the eluted factor samples as well as half life of the expensive column material.

Thus, different to currently commercially available recombinant FVIII preparations the present invention is directed to a next generation product that is produced in culture media devoid of any components of human or animal origin using compounds and methods described herein. Furthermore, the present invention may also be directed to purification of plasma-derived FVIII preparations.

The present invention includes compounds comprising chemically synthesized unique high-affinity peptides and peptide derivatives which can replace monoclonal antibodies and have improved proteolytic stability compared to the known oligopeptides mentioned above. Furthermore, the inventive compounds are suitable for large scale solution synthesis and therefore will minimize the production costs.

### SUMMARY OF THE INVENTION

The present invention is directed to specific compounds comprising peptides and peptide derivatives. These compounds exhibit particular properties of binding and/or releasing FVIII or FVIII-like polypeptides or domains thereof, wherein the domains are substantially as defined above for the FVIII-molecule, and may serve as ligands for affinity separation of FVIII, FVIII-like polypeptides or domains thereof.

In specific embodiments the compounds of the present invention comprising peptides and peptide derivatives are tetrapeptides, pentapeptides, hexapeptides and heptapeptides that bind FVIII or FVIII-like proteins or domains thereof with affinity sufficient for chromatographic purification of FVIII.

In certain embodiments, the compounds are binding molecules that exhibit distinct characteristics for binding of the target Factor VIII polypeptides as well as specific characteristics for release (elution) of the target polypeptides (i.e. specific composition and pH of application and elution buffers). To facilitate elution of the product under mild conditions, the compounds may easily be modified by existing chemical methods. Such modification is not technically feasible for the conventionally used antibodies.

A further embodiment relates to an inert matrix as solid carrier material comprising the immobilized compound, preferably a peptide or peptide derivative. In specific embodiments, the solid carrier material is a polymeric material. In further specific embodiments, the compound is chemically bound to the solid carrier matrix. In another specific embodiment, the compound is chemically bound to the solid carrier matrix via an anchoring molecule. In a further specific embodiment, the compound is chemically bound to the solid carrier matrix via a spacer molecule. It is also contemplated that the compound is chemically bound to the solid carrier matrix through an anchoring molecule and an additional spacer molecule.

The present invention relates to a diagnostic device or kit comprising a compound of the present invention immobilized on a matrix, wherein the compound binds specifically to a FVIII or FVIII-related protein or domains thereof. In certain embodiments, the compound is directly or via an anchoring compound and/or a spacer molecule immobilized on the matrix, which may be a polymeric material such as, for example, a resin.

In yet another embodiment, the compounds are used in methods as a label of a FVIII or FVIII-like protein or domains thereof.

In an embodiment of the present invention, the compound is used in methods of identification and/or purification of FVIII or FVIII-like proteins or domains thereof.

In another embodiment of the present invention, the compound is used in methods to stabilize FVIII or FVIII-like proteins or domains thereof, substantially as defined above for the FVIII-molecule.

In yet another embodiment of the present invention, the compound is used in methods to enhance the activity of FVIII or FVIII-like proteins.

The present invention relates further to the medical use of the compound of the present invention in the treatment of diseases.

### DETAILED DESCRIPTION OF THE INVENTION

The affinity chromatography is a well established powerful technique which is a state-of-the-art procedure used for purification of complex molecules such as proteins (Jack, G. W.; Beer, D. J. Methods Mol. Biol. 1996, 59, 187-196). Affinity chromatography offers the unique possibility to isolate the target protein with excellent selectivity from contaminating proteins by its strong interaction between a target molecule and a ligand, which is immobilized on a resin. Usually, the ligands are either polyclonal or monoclonal antibodies. Monoclonal antibodies are preferred, because they are monospecific and can be produced with precision (Scopes, R. K. Protein purification: Principles and Practice. Springer, New York, 1994). Small chemical ligands had so far only limited application in affinity separation. However, the use of combinatorial libraries has expanded the repertoire of immunoaffinity chromatography techniques for peptide ligands (Lowe, C. R. Curr. Opin. Chem. Biol. 2001, 5, 248-256). Based on either biological or chemical systems the use of the combinatorial methods have generated unique peptides which provide moderate or even high binding affinity to capture the target protein and elute it under mild conditions.

Huang, Ping Y. et al., for example, describe in "Bioorganic & Medicinal Chemistry", Vol. 4, No. 5, pp. 699-708, 1996 the use of immobilized peptides for the immunoaffinity chromatography purification of von Willebrand Factor (vWF). Purification of multimeric vWF is a major challenge because the molecular weight ranges between the enormous size of 0.5 to 10 million Daltons. The von Willebrand Factor is a multifunctional plasma protein directly involved in the blood coagulation cascade. It has a prominent role in the events that lead to normal arrest of bleeding. Interactions between vWF and FVIII result in stabilizing and transporting FVIII. Two high affinity binding sites ensure efficient capture (Sadler. J. E.; Mannucci. P. M.; Bemtorp. E.; Bochkov. N.; Boulyjenkov. V.; Ginsburg. D.; Meyer. D.; Peake. I.; Rodeghiero. F.; Srivastava. A. Thromb. Haemost., 2000, 84, 160-174).

FVIII is a large and complex protein which plays an important function in the blood coagulation cascade and has great therapeutic significance. Deficiencies in FVIII production *in vivo* caused by genetic mutations can lead to hemophilia which is treated by infusion of purified preparations of human FVIII (Lee, C. Thromb. Haemost. 1999, 82, 516-524). The current sources of human FVIII for treatment of hemophilic patients are plasma-derived FVIII and recombinant FVIII, the latter synthesized in Chinese hamster ovary (CHO) cells (Kaufman, R. J.; Wasley, L. C.; Domer, A. J. J. Biol. Chem. 1988, 263, 6352-6362) and baby hamster kidney (BHK) cells (Boedeker, B. D. G. Sem. Thromb. Hemost. 2001, 27, 385-394). In addition to high purity criteria, it is critical to ensure immunological and virus safety.

There are several methods known for purification of human FVIII using chromatographic techniques. The use of an immunoaffinity chromatography resin is a common manufacturing procedure for all recombinant FVIII preparations, and for many plasma-derived FVIII products. The current manufacturing process which includes affinity chromatography uses a monoclonal antibody (mAb) that is specific for FVIII (Lee, C., Recombinant clotting factors in the treatment of hemophilia. Thromb. Haemost. 1999, 82, 516-524). For manufacturing purposes the antibody against FVIII is produced by a murine hybridoma cell line and then immobilized to a chromatographic resin. The current industrial FVIII purification utilizes a mAb immunoaffinity step providing excellent removal of process-related impurities such as DNA and host cells.

However, there are a variety of concerns and limitations connected with the current process of immunoaffinity chromatography using immobilized monoclonal antibodies. One disadvantage is a limitation in the capacity of the resin because a few antibody molecules, huge in molecular size, will cover a considerable part of the resin surface. In addition, the antibody preparation is a lengthy and expensive method, and the purity and activity of the antibodies vary depending on each iterative preparation. The antibodies are produced by a hybridoma culture as the production host which makes the antibodies susceptible to a low, but non-zero risk that viruses, especially retroviruses, may be introduced into the manufacturing process of the target protein. Finally, the leakage of the antibodies from the solid carrier matrix, i.e. the resin, can lead to serious product contaminations and result in the loss of the product due to immunogenicity. Thus, there is sufficient motivation to replace the current process by a more precise, cost-effective process. The present invention fulfills this need by providing a compound which is a chemically synthesized small peptide in an immunoaffinity chromatography purification method that offers a reduction or elimination of several of the described pitfalls.

Pflegerl et al. (J. Peptide Res. 2002, 59, 174-182) reported the development of different octapeptides with high affinity towards FVIII. The essential amino acid sequence was found to be WEY, located in the C-terminal side of the peptides.

The compounds of this invention comprising small peptides and peptide derivatives have advantages as ligands, because they are unlikely to provoke immune responses in case of leakage into the product. Small peptides and peptide derivatives are also much more stable in comparison with antibodies. Another advantage is their significant lower production costs, since they can easily be manufactured aseptically in huge quantities under good manufacturing practices (GMP). The use of the small peptides and peptide derivatives and methods of the present invention achieve a purified product using no animal-derived or human-derived raw materials. Last but not least, the sophisticated chemical synthesis described herein allows refined steps to improve the affinity of the small peptides and peptide derivatives towards their target protein.

Therefore, the present invention provides the ordinary artisan working in the field with a compound and a process that improves the commonly used purification procedure of FVIII.

As described herein, the present invention provides a FVIII purification method that avoids the use of mouse monoclonal antibodies for immunoaffinity purification of FVIII. The invention includes chemically synthesized unique high-affinity peptides and peptide derivatives which can replace monoclonal antibodies and have improved proteolytic stability compared to the known oligopeptides mentioned above. This would meet the up-to-date requirements for biological safety. Furthermore, the inventive compounds are suitable for large scale solution synthesis and therefore minimize the production costs of the affinity ligands.

The present invention comprises novel compounds, preferably pentapeptides as ligands for detecting, identifying, isolating and purifying as well as labeling active Factor VIII, Factor VIII-like proteins or domains thereof from solutions that contain such proteins. The Factor VIII binding molecules of the present invention exhibit remarkable stability as well as high affinity for Factor VIII, Factor VIII-like peptides or domains thereof. The invention provides a cost-effective means to ensure fast separation and purification of commercial quantities of proteins useful in the treatment of hemophilia A.

The invention relates to compounds comprising peptides and peptide derivatives of formula I

B-Q-X (I)

wherein
- B: is a tetrapeptide, pentapeptide, hexapeptide or heptapeptide binding to FVIII and/or FVIII-like proteins or domains thereof,
- Q: is absent or an organic spacer molecule and
- X: is absent or an organic anchoring molecule,
as well as their pharmaceutically acceptable salts.

Preferably, the peptides according to the present invention bind via an affinity towards FVIII and/or FVIII-like proteins or domains thereof. "Affinity" is the force of attraction between atoms or molecules that helps to keep them in combination. This is the basis for affinity chromatography. Affinity can also be defined as a measure of the intrinsic binding strength of the ligand binding reaction. The intrinsic attractiveness of the binder for the ligand is typically expressed as the equilibrium constant (Ka) of the reaction. The equilibrium constant Ka = [Ligand-Binder]/[Ligand][Binder], where [ ] represents the molar concentration of the material at equilibrium.

The peptide or peptide derivative B of the present invention will be chemically bound to the surface of the preferred embodied solid carrier matrix, preferably, with the help of an anchorage molecule X and/or a spacer molecule Q or, if Q and X are missing, preferably by a SH, N₃, NH-NH₂, O-NH₂, NH₂, -CH₂-L, C≡CH, carbonyl or carboxyl group of the compound B. Herein L comprises a leaving group, like Cl, Br or I.

The term "chemical binding" includes covalent, ionic, hydrophobic and/or other complex interactions, as well as mixtures and combinations thereof, between two (or more) atoms, or one (or more) atom(s) and one (or more) compound(s), or, two (or more) compounds.

In one preferred embodiment,
- B: is E¹HN-(Z)ₙ-COE²
wherein
- E¹: is H, R¹, -COR¹ or -CO₂R¹-,
- E²: is -OR² or -NHR², or NH-NHR²
wherein
- R¹: is C1-4 alkyl, Ar or CH₂-Ar,
- R²: is H or R¹,
- Ar: is an unsubstituted phenyl; or is a substituted phenyl which is one-, two-, or threefold substituted with A, OH, OA, CF₃, OCF₃, CN, NO₂ or Hal, which can be substituted one-, two-, or threefold with an A, OH, OA, CF₃, OCF₃, CN, NO₂ or Hal substituted phenyl, in such a way that an unsubstituted or substituted biphenyl is created; or is Het,
wherein Hal is selected from F, Cl, Br or I,
Het is a saturated or unsaturated mono- or bicyclic heterocyclic residue with 5 to 12 ring members, comprising 1 to 3 N- and/or 1 S- or O- atoms, wherein the heterocyclic residue can be substituted one- or two-fold with CN, Hal, OH , NH₂, COOH, OA, CF₃, A, NO₂, Ar or OCF₃,
A is COOH, NH₂ or alkyl with 1-6 C-atoms, unsubstituted or substituted with COOH or NH₂,
- Z: is a naturally occurring or non-proteinogenic amino acid residue or derivative thereof,
- n: is an integer between 4 and 7,
wherein
Z can be linked by a bond selected from the group of an acid-amide bond - CO-NR³- or -NR³-CO-, a reduced peptide bond -CH₂-NR³- or -NR³-CH₂-, a bond -CO-CHR³-, -CHR³-CO-, -CR³=CH- or a -CH=CR³- bond,
wherein R³ is selected from H, C₁₋₄alkyl, phenyl or benzyl or, in case of peptoid-amino acids, the amino acid side chain,
and pharmaceutically acceptable salts thereof.

In an embodiment Q is an organic spacer molecule selected from one of the following groups
[-NH-(CH₂)ₓ-CO]_{w},
[-NH-(CH₂CH₂-O-)_{y}CH₂-CO]_{w},
[CO-(CH₂)_{z}-CO-],
[NH-(CH₂)_{z}-NH-],
[CO-CH₂-(OCH₂CH₂)_{y}-O-CH₂-CO-],
[NH-CH₂CH₂-(OCH₂CH₂)_{y}-NH-],
as well as their combinations
wherein
- w: is an integer from between 1 to 8,
- x: is an integer from between 1 to 5,
- y: is an integer from between 1 to 6, and
- z: is an integer from between 1 to 6.
and pharmaceutically acceptable salts thereof.
Organic spacer molecules are known per se. "Organic" refers to all carbon compounds except carbide and carbonate compounds, see also Beilstein's Handbook of Organic Chemistry. Usually, the organic spacer molecule is a linear hydrocarbon having a functional groups at one or both terminal ends. The hydrocarbon chain can be modified.

In another embodiment X is an organic anchoring molecule selected from one of the following groups
a naturally occurring or non-proteinogenic amino acid,
-A¹-(CH₂)ₚ-A²,
-A¹-CH₂-(OCH₂CH₂)_{y}-O-CH₂-A²,
-A¹-CH₂CH₂-(OCH₂CH₂)_{y}-A²,
=CR²-(CH₂)ₚ-A²,
-A¹-CH(NHR⁴)-(CH₂)_{q}-A²,
=CR²-CH(NHR⁴)-(CH₂)_{q}-A²,
-A¹-CH(COR⁵)-(CH₂)_{q}-A² or
=CR²-CH(COR⁵)-(CH₂)_{q}-A²,
wherein
- A¹: is NR², CO, CHR², O, or S,

- A²: is SH, N₃, NH-NH₂, O-NH₂, NH₂, Hal¹, C≡CH, CR⁶O, or carboxyl,
- R²: is as defined before,
- R⁴: is H, R⁶, -COR⁶, -COOR⁶,
- R⁵: is -OR⁶ or NHR⁴,
- R⁶: is H, C₁₋₄alkyl; or is unsubstituted phenyl or with A, OH, OA, CF₃, OCF₃, CN, NO₂ or Hal one-, two-, or threefold substituted phenyl; or benzyl,
- p: is an integer from between 1 to 20,
- q: is an integer from between 1 to 20,
- y: is an integer from between 1 to 6,
- z: is an integer from between 1 to 6,
- Hal: is an integer from between as defined above in Claim 2, and
- Hal¹: is Cl, Br or I.

"Anchoring molecules" are molecules or molecule-groups which can be applied for linking fragments (i.e. a compound and a resin). Such anchoring molecules are known per se. Usually anchoring molecules comprise two or more functional groups which can form a chemical binding.

Another embodiment is characterized that at least one residue Z from (Z)ₙ with n between 4 to 7 is selected from alanine, valine, serine, threonine or α-aminobutyric acid side chain.

A further embodiment is characterized that at least one residue Z from (Z)ₙ with n between 4 to 7 is selected from cysteine, homo-cysteine or D-cysteine.

Another embodiment is characterized that at least one residue Z from (Z)ₙ with n between 4 to 7 is selected from glutamic or aspartic side chain.

Another embodiment is further characterized that at least one residue Z from (Z)ₙ with n between 4 to 7 is selected from phenylalanine, tyrosine, O-methylated tyrosine, 1-naphthylalanine, 2-naphthylalanine, tryptophane, p-benzoylphenylalanine or -CH₂-Ar side chain, wherein Ar is as defined above.

An embodiment is further characterized that E¹ is H or acetyl.

Another embodiment is further characterized that E² is OH or NH₂.

Another embodiment is further characterized that Q is [-NH-(CH₂)ₓ-CO]_{w} with x and w are as defined before.

An embodiment of the invention comprises compounds further characterized that x is 1 or 5 and w is 0 or 1.

Another embodiment of the invention comprises compounds further characterized that
X is -A¹-CH(COR⁵)-(CH₂)_{q}-A²
wherein
- A¹: is NH
- R⁵: is OH or NH₂
- A²: is SH
and q is as defined before.
A preferred embodiment of the invention comprises compounds further characterized that q is 1.

Another embodiment of the invention comprises compounds further characterized that
X is -A¹-CH(NHR⁴)- (CH₂)_{q}-A²
wherein
- A¹: is CO
- R⁴: is H
- A²: is SH
and q is as defined before.

A preferred embodiment of the invention comprises compounds further characterized that q is 1.

Another embodiment of the invention comprises compounds further characterized that the amino acid residues can be independently chosen from L- or D-α-amino carbonic acid residues, β-amino carbonic acid residues, aza-amino carbonic acid residues and peptoid-amino carbonic acid residues.

A preferred embodiment of the invention comprises compounds further characterized that the amino acid residues are chosen from α-amino acid residues.

Another embodiment comprises compounds further characterized that at least two of the residues Z are bound by acid-amide bonds -CO-NH- or -NH-CO-.

A more preferred embodiment of the invention comprises compounds further characterized that at least two of the residues Z are bound by N-methylated acid-amide bonds -CO-NCH₃-or-NCH₃-CO-.

Another embodiment of the invention comprises compounds further characterized that one or more peptide bonds can be independently modified.

A further embodiment of the invention comprises compounds further characterized that at least two of the residues Z are bound by -CH₂-NH- or -NH-CH₂- bonds.

Another embodiment of the invention comprises compounds further characterized that the direction of the peptide sequence is inverted ("retropeptide").

Another embodiment of the invention comprises compounds further characterized that the direction of the peptide sequence is inverted and the amino acid residues are chosen from D-α-amino acid residues ("retro-inverso-peptide").

An embodiment of the invention comprises a pharmaceutical composition, wherein the compound is selected from the group

| | |
|---|---|
| S1 | **Y-C-S-W-E-Y-NH₂** |
| S2 | **Ac-Y-C-S-W-E-Y-NH₂** |
| S3 | **Y-C-T-W-D-Y-NH₂** |
| S4 | **Ac-Y-C-T-W-D-Y-NH₂** |
| S5 | **Y-*homo*C-S-W-E-Y** |
| S6 | **Y-c-S-W-E-Y** |
| S7 | **Y-C-S-W-E-Y** |
| S8 | **y-e-w-s-c-y** |
| S9 | **Ac-Y-C-S-W-E-Y** |
| S10 | **Y-C-T-W-E-Y** |
| S11 | **Y-C-S-W-D-Y** |
| S12 | **Y-C-T-W-D-Y** |
| S13 | **y-d-w-t-c-y** |
| S14 | **Ac-Y-C-T-W-D-Y** |
| S15 | **Y-C-S-Bpa-E-Y** |
| S16 | **Y-C-A-W-D-Y** |
| S17 | **y-d-w-a-c-y** |
| S18 | **Y-C-V-W-D-Y** |
| S19 | **y-d-w-v-c-y** |
| S20 | **C-S-W-E-Y** |
| S21 | **(*N*-Me)S-W-E-Y-***C* |
| S22 | **Y-F-(*N*-Me)S-W-E-Y-***C* |
| S23 | **Y-F-S-W-(*N*-Me)E-Y-***C* |
| S24 | **Y-C-(*N*-Me)S-W-E-Y** |
| S25 | **Y-C-S-W-(*N*-Me)E-Y** |
| S26 | **Y-C-S-W-E-(*N*-Me)Y** |
| S27 | **Y-f-(*N*-Me)S-W-E-Y-***C* |
| S28 | **Y-f*-*S*-*W*-*(*N*-Me)E-Y-***C* |
| S29 | **H-S-W-E-Y-***C* |
| S30 | **Ac-S-W-E-Y-***C* |
| S31 | **Ac-H-S-W-E-Y-***C* |
| S32 | **Ac-Y-H-S-W-E-Y-***C* |
| S33 | **Y-F-Abu-W-E-F-***C* |
| S34 | **Y-f-S-W-E-Y-***C* |
| S35 | **c-y-e-w-s** |
| S36 | **Y-F-Abu-W-E-Y-***C* |
| S37 | **Y-H-S-Bpa-E-Y-***C* |
| S38 | **Y-H-S-W-E-Bpa-***C* |
| S39 | **Y-H-Abu-W-D-Y-***C* |
| S40 | **Y-S-W-E-Y-***C* |
| S42 | **Y-H-S-(2-Nal)-E-Y-***C* |
| S43 | **S-W-D-Y-***C* |
| S44 | **E-S-W-E-Y-***C* |
| S45 | **Ac-E-S-W-E-Y-***C* |
| S46 | **T-W-E-Y-***C* |
| S47 | **Y-H-T-W-E-Y-***C* |
| S48 | **Y-H-S-W-D-Y-***C* |
| S49 | **Y-F-Abu-W-E-Y-***C* |
| S50 | **Abu-W-E-Y-***C* |
| S51 | **Y-H-S-Bpa-E-Bpa-***C* |
| S52 | **Y-H-T-W-D-Y-***C* |
| S53 | **S-W-E-Phg-C** |
| S54 | **Y-H-Abu-W-E-Y-***C* |
| S55 | **Y-H-S-W-E-Bip(4,4')-***C* |
| S56 | **Y-H-S-W-E-(2-Nal)-***C* |
| S57 | **S-Bpa-E-(1-Nal)-***C* |
| S58 | **S-Bpa-E-(2-Nal)-*C*** |
| S59 | **S-Bpa-E-Bip(4,4')-*C*** |
| S60 | **S-Bpa-E-Phe(4-Cl)-***C* |
| S61 | **S-Bpa-E-Bpa-C** |
| S62 | **S-(1-Nal)-E-Bip(4,4')-***C* |
| S63 | **S-(2-Nal)-E-Bip(4,4')-***C* |
| S64 | **S-Bip(4,4')-E-Bpa-C** |
| S65 | **S-Bta-E-Bpa-C** |
| S66 | **S-Phe(4-Cl)-E-Bpa-*C*** |
| S67 | **S-Phe(3,4-Cl)-E-Bpa-***C* |
| S68 | **Y-Y-S-W-E-Y-***C* |
| S69 | **(*N*-Me)Y-C-S-W-E-Y** |
| S70 | **Y-C-S-(*N*-Me)W-E-Y** |
| S71 | **S-Bpa-E-Y-***C* |
| S72 | **S-Bpa-E-Phg-C** |
| S73 | **S-W-E-Bpa-***C* |
| S74 | **Y-H-S-W-E-Phg-***C* |
| S75 | **Y-H-S-Bpa-E-Phg-***C* |
| S76 | **Y-H-V-W-E-Y-***C* |
| S77 | **Y-H-S-Phe(3,4-Cl)-E-Y-***C* |
| S78 | **Y-H-S-Bta-E-Y-***C* |
| S79 | **Y-W-E-Y-***C* |

together with a suitable carrier, wherein, as used herein, the binding molecules are marked in bold, linker are marked in italics and small letters represent D-amino acids.

Another embodiment of the invention comprises a compound for the treatment of diseases.

Another embodiment of the invention can be bound to a solid carrier matrix with a surface.

The solid carrier matrix to which an embodiment of the invention is bound can comprise inorganic or organic, especially polymeric material.

Another embodiment of the invention is bound to a solid carrier matrix wherein the compound or the compounds are chemically bound to the surface of the solid carrier matrix, for example to a resin.

A preferred embodiment of the invention is bound to a solid carrier matrix wherein the compound or the compounds are bound through organic spacers to the surface of the solid carrier matrix, for example to a resin.

Another preferred embodiment of the invention is bound to a solid carrier matrix wherein the compound or the compounds are bound through an organic anchoring molecule to the surface of the solid carrier matrix, for example to a resin.

A further preferred embodiment of the invention is bound to a solid carrier matrix wherein the compound or the compounds are bound through organic spacers and an organic anchoring molecule to the surface of the solid carrier matrix, for example to a resin.

The solid carrier material comprises inorganic or organic, especially polymeric, material. Therefore, the same polymeric material (i.e. linear polysaccharide) can be utilized which is usually employed for the chromatography of biopolymers. In particular, polymers exerting a hydrophilic surface are suitable as a chromatography solid carrier material, i.e. a resin. For example, the Toyopearl AF-Epoxy-650M resin is employed.

Such solid carrier material can also be provided with an additional anchoring molecule offering, for example, a SH, N₃, NH-NH₂, O-NH₂, NH₂, -CH₂-L, C≡CH, epoxy, carbonyl or carboxyl group for immobilization of compounds like peptides and peptide derivatives according to formula I.

Due to the fact that the preferred compounds of the current invention interact with FVIII, FVIII-like proteins and/or domains thereof, the preferred compounds comprising peptides and peptide derivatives are suitable for diagnostic devices and kits. The preferred diagnostic device or kit comprises at least one compound, having a high affinity for FVIII or FVIII-like proteins or domains thereof, a solid carrier matrix to which at least one compound may optionally be bound chemically, and other reagents, if needed.

In order to follow the results of a reaction of the compound, preferably a peptide or peptide derivative, of the present invention with FVIII, FVIII-like proteins and/or domains thereof, the compound is labeled. There are several possibilities to label the compound, i.e. by using radioactive markers, by using fluorescent ligands, by using the avidine/streptavidine system, or, as is common in the ELISA technique, by using enzymes which provoke color reactions.

The invention comprises further a method of detecting, identifying, diagnosing, isolating, purifying, stabilizing and/or enhancing the activity of FVIII or FVIII-like proteins or domains thereof comprising contacting a sample comprising a FVIII or FVIII-like protein or domains thereof with a matrix comprising an immobilized compound, under conditions suitable for binding between FVIII or FVIII-like protein or domains thereof and said compound.

In another method the compound is immobilized on a solid carrier and the solid carrier is a polymeric material.

In a preferred method the compound is selected from the group of S1, S2, S3, S4, S5, S6, S7, S8, S9, S10, S11, S12, S13, S14, S15, S16, S17, S18, S19, S20, S21, S22, S23, S24, S25, S26, S27, S28, S29, S30, S31, S32, S33, S34, S35, S36, S37, S38, S39, S40, S41, S42, S43, S44, S45, S46, S47, S48, S49, S50, S51, S52, S53, S54, S55, S56, S57, S58, S59, S60, S61, S62,S63,S64,S65,S66,S67,S68,S69,S70,S71,S72,S73,S74,S75,S76,S77,S78 or S79, as defined above.

In a further method the polymeric material is a resin. In another method the sample is a cell culture supernatant. In another preferred method the sample is a body fluid. In a more preferred method the body fluid comprises serum or whole blood.

Another embodiment of the invention comprises a method for producing a FVIII or FVIII-like protein or domains thereof containing a pharmaceutical composition or medicament, comprising a method for purifying a FVIII or FVIII-like protein or domains thereof and formulating the purified FVIII or FVIII-like protein or domains thereof.

Yet another embodiment of the invention comprises a method for producing and/or stabilizing a FVIII or FVIII-like protein or domains thereof containing a pharmaceutical composition or medicament, comprising a method for purifying and/or stabilizing a FVIII or FVIII-like protein or domains thereof and formulating said purified and/or stabilized FVIII or FVIII-like protein or domains thereof.

Another embodiment of the invention comprises a method for enhancing the activity of a FVIII or FVIII-like protein or their domain containing a pharmaceutical composition or medicament, comprising a method for enhancing the activity of a FVIII or FVIII-like protein or domains thereof and formulating said activity-enhanced FVIII or FVIII-like protein or domains thereof.

Preliminary experiments show that the addition of the compounds according to the invention leads to a stabilization of the FVIII or FVIII-like protein or domains thereof and/or enhancement of the activity of the FVIII or FVIII-like protein or domains thereof

In another embodiment the compound is used for labeling, detecting, diagnosing, monitoring, identifying, isolating, purifying, stabilizing and enhancing the activity of a FVIII or FVIII-like protein or domains thereof.

In order to follow the results of a reaction of the compound, preferably a peptide or peptide derivative, of the present invention with FVIII, FVIII-like proteins and/or domains thereof, the compound is labeled. There are several possibilities to label the compound, i.e. by using radioactive markers, by using fluorescent ligands, by using the avidine/streptavidine system, or, as is common in the ELISA technique, by using enzymes which provoke color reactions.

The molecules of the present invention relates to compounds comprising peptides and peptide derivatives which are suitable for labeling, detecting, identifying, isolating, purifying, stabilizing and/or enhancing the activity of FVIII and/or FVIII-like proteins and/or domains thereof. Preliminary experiments in the context of the present invention showed that the addition of at least one of the peptides according to the invention leads to a stabilization of the activity of FVIII and/or FVIII-like proteins and/or domains thereof or enhancement of the activity of FVIII and/or FVIII-like proteins and/or domains thereof in a standard chromogenic assay by 10%, preferably by 30%.

The abbreviations of amino acids given above and below stand for the residues of the following amino acids:

| | |
|---|---|
| Abu | 4-Aminobutyric acid |
| Aha | 6-Aminohexanoic acid |
| Ala | Alanine |
| Asn | Asparagine |
| Asp | Aspartic acid |
| Arg | Arginine |
| Bip(4, 4') | 4,4'-Biphenylalanine |
| Bpa | p-Benzoylphenylalanine |
| Bta | Benzothienylalanine |
| Cys | Cysteine |
| Dab | 2,4-Diaminobutyric acid |
| Dap | 2,3-Diaminopropionic acid |
| Gln | Glutamine |
| Glp | Pyroglutamic acid |
| Glu | Glutamic acid |
| Gly | Glycine |
| His | Histidine |
| *homo-Cys* | homo-Cysteine |
| *homo-Phe* | homo-Phenylalanine |
| IAA | 2-(Indol-3-yl)acetic acid |
| IBA | 4-(Indol-3-yl)butyric acid |
| IPA | 3-(Indol-3-yl)propionic acid |
| Ile | Isoleucine |
| Leu | Leucine |
| Lys | Lysine |
| Met | Methionine |
| 1-Nal | 1-Naphthylalanine |
| 2-Nal | 2-Naphthylalanine |
| Nle | Norleucine |
| Orn | Ornithine |
| Phe | Phenylalanine |
| Phe(4-Cl) | 4-Chlorophenylalanine |
| Phe(3,4-Cl) | 3,4-Dichlorephenylalanine |
| Phg | Phenylglycine |
| 4-Hal-Phe | 4-Halogen-phenylalanine |
| Pro | Proline |
| Ser | Serine |
| Thr | Threonine |
| Trp | Tryptophan |
| Tyr | Tyrosine |
| Val | Valine |

Furthermore, the following abbreviations are used below:

| | |
|---|---|
| Ac | Acetyl |
| BOC | *tert*-Butoxycarbonyl |
| tBu | *tert*-Butyl |
| CBZ oder Z | Benzyloxycarbonyl |
| DCCI | Dicyclohexylcarbodiimide |
| DIPEA | *N*-Ethyldiisopropylethylamine |
| DMF | Dimethylformamide |
| EDCI | *N*-Ethyl-*N*,*N*'-(dimethylaminopropyl)-carbodiimide |
| Et | Ethyl |
| Fmoc | 9-Fluorenylmethoxycarbonyl |
| HOBt | 1-Hydroxybenzotriazole |
| Me | Methyl |
| MBHA | 4-Methyl-benzhydrylamine |
| Mtr | 4-Methoxy-2,3,6-trimethylphenyl-sulfonyl |
| HATU | O-(7-Azabenzotriazol-1-yl)-N,N,N',N'-Tetramethyluronium-hexafluorophosphate |
| HONSu | *N*-Hydroxysuccinimide |
| OtBu | *tert*-Butylester |
| Oct | Octanoyl |
| OMe | Methylester |
| OEt | Ethylester |
| POA | Phenoxyacetyl |
| Pbf | Pentamethylbenzofuranyl |
| Pmc | 2,2,5,7,8-Pentamethylchroman-6-sulfonyl |
| Sal | Salicyloyl |
| Su | Succinyl |
| TIPS | Triisopropylsilane |
| TFA | Trifluoracetic acid |
| TFE | Trimethylsilylbromide |
| Trt | Trityl (Triphenylmethyl). |

If the building blocks of the compounds of the formula I (i.e. the amino acids mentioned above) can occur in a plurality of enantiomeric forms, all these forms and also mixtures thereof (for example the DL forms) are included.

Furthermore, the amino acids may, for example, as a constituent of compounds of the formula I, be provided with corresponding protecting groups known per se. Favored groups are derivatives of Asp and Glu, particularly methyl-, ethyl-, propyl-, butyl-, *tert*-butyl-, neopentyl-or benzylester of the side chain or derivatives of Tyr, particularly methyl-, ethyl-, propyl-, butyl-, tert-butyl-, neopentyl- or benzylethers of the side chain.

In addition, also structural elements like *N*-terminal modified or carboxy-terminal modified derivatives are part of this invention. Favored groups are amino-terminal methyl-, ethyl-, propyl-, butyl-, *tert*-butyl-, neopentyl-, phenyl- or benzyl-groups, amino-terminal groups like BOC, Mtr, CBZ, Fmoc, and, particularly, acetyl, benzoyl or (indol-3-yl)carbonic acid groups, furthermore, carboxy-terminal methyl-, ethyl-, propyl, butyl-, tert-butyl-, neopentyl- or benzylester, methyl-, ethyl-, propyl-, butyl-, tert-butyl-, neopentyl- or benzylamides and, particularly, carboxamides.

Amino acids, which can be used for the formation of the peptides and peptide derivatives according to the present invention, can belong to both natural occurring and non-proteinogenic amino acids. Amino acids and amino acid residues can be derivatized, whereas *N*-methyl-, *N*-ethyl-, N-propyl- or *N*-benzyl- derivatives are favored. For example, if a methyl is employed, the *N*-alkylation of the amide binding can have a strong influence on the activity of the corresponding compound (Levian-Teitelbaum, D.; Kolodny, N.; Chorev, M.; Selinger, Z.; Gilon, C. Biopolymers 1989, 28, 51-64 which is herein incorporated by reference in its entirety). Further structural alternatives of amino acids that can be used include amino acids with modifications in the side chain, β-amino acids, aza-amino acids (derivatives of α-amino acids, where the α-CH-group is substituted by a N-atom) and/or peptoid-amino acids (derivatives of α-amino acids, where the amino acid side chain is bound to the amino group instead to the α-C-atom) or cyclised derivatives from the above mentioned modifications.

The invention furthermore relates to the process for the preparation of compounds of the formula I and salts thereof. It is contemplated that structural elements like N-terminal modified or carboxy-terminal modified derivatives are part of this invention.

The compounds of formula I can have one or more centers of chirality and can therefore occur in various stereoisomeric forms.

Accordingly, the invention relates in particular to the compounds of the formula I in which at least one of the said residues is mentioned as preferred.

**Table 1 : Particularly preference is given to the following compounds of the formula I (as used here, the binding molecules are marked in bold, linker are marked in italics and small letters represent D-amino acids):**

| Label | Sequence | % Binding pdFVIII | % Binding ReFacto | Loading density (µmol/mL) |
|---|---|---|---|---|
| **S1** | **Y-C-S-W-E-Y-NH₂** | 41,8 ± 3,2 | | 15,9 |
| **S2** | **Ac-Y-C-S-W-E-Y-NH₂** | 59,2 ± 4,6 | | 16,6 |
| **S3** | **Y-C-T-W-D-Y-NH₂** | 42,3 ± 4,3 | | 16,3 |
| **S4** | **Ac-Y-C-T-W-D-Y-NH₂** | 58,5 ± 2,4 | | 16,1 |
| **S5** | **Y-*homo*C-S-W-E-Y** | 59,7 ± 3,8 | | 15,2 |
| **S6** | **Y-c-S-W-E-Y** | 55,8 ± 2,3 | | 15,1 |
| **S7** | **Y-C-S-W-E-Y** | 64,1 ± 3,2 | | 15,5 |
| **S8** | **y-e-w-s-c-y** | 69,4 ± 6,6 | | 14,5 |
| **S9** | **Ac-Y-C-S-W-E-Y** | 81,6 ± 2,8 | | 14,2 |
| **S10** | **Y-C-T-W-E-Y** | 80,3 ±1,3 | | 12,1 |
| **S11** | **Y-C-S-W-D-Y** | 79,9 ±4,0 | | 14,3 |
| **S12** | **Y-C-T-W-D-Y** | 74,2 ±4,2 | | 15,7 |
| **S13** | **y-d-w-t-c-y** | 53,8 ±0,5 | | 15,9 |
| **S14** | **Ac-Y-C-T-W-D-Y** | 82,3 ±1,4 | | 16,4 |
| **S15** | **Y-C-S-Bpa-E-Y** | 49,4 ±2,2 | | 17,7 |
| **S16** | **Y-C-A-W-D-Y** | 72,0 ±15,5 | | 17,2 |
| **S17** | **y-d-w-a-c-y** | 75,1 ± 0,6 | | 16,0 |
| **S18** | **Y-C-V-W-D-Y** | 72,0 ±1,2 | | 14,7 |
| **S19** | **y-d-w-v-c-y** | 73,4 ±5,6 | | 16,5 |
| **S20** | **C-S-W-E-Y** | 48,6 ± 3,3 | 45,3 ± 3,1 | |
| **S21** | **(*N*-Me)S-W-E-Y-***C* | 48,4 ± 3,7 | | 13,2 |
| **S22** | **Y-F-(*N*-Me)S-W-E-Y-***C* | 47,6 ± 4,6 | | 14,7 |
| **S23** | **Y-F-S-W-(*N*-Me)E-Y-***C* | 53,1 ± 0,3 | | 14,7 |
| **S24** | **Y-C-(*N*-Me)S-W-E-Y** | 14,4 ±0,4 | | 11,3 |
| **S25** | **Y-C-S-W-(*N*-Me)E-Y** | 45,2 ±2,5 | | 13,8 |
| **S26** | **Y-C-S-W-E-(*N*-Me)Y** | 48,8 ±3,1 | | 13,9 |
| **S27** | **Y-f-(*N*-Me)S-W-E-Y-***C* | 57,5 ±4,6 | | 12,6 |
| **S28** | **Y-f-S-W-(*N*-Me)E-Y-***C* | 65,1 ±2,5 | | 14,3 |
| **S29** | **H-S-W-E-Y-***C* | 54,4 ±4,9 | | 14,3 |
| **S30** | **Ac-S-W-E-Y-***C* | 42,6 ± 1,3 | | 12,2 |
| **S31** | **Ac-H-S-W-E-Y-***C* | 48,9 ±3,0 | | 11,0 |
| **S32** | **Ac-Y-H-S-W-E-Y-***C* | 62,2 ± 4,4 | | 12,8 |
| **S33** | **Y-F-Abu-W-E-F-***C* | 53,4 ± 5,3 | | 15,2 |
| **S34** | **Y-f-S-W-E-Y-***C* | 53,6 ± 2,7 | | 15,4 |
| **S35** | **c-y-e-w-s** | 37,1 ± 1,0 | | 9,2 |
| **S36** | **Y-F-Abu-W-E-Y-***C* | 62,0 ± 4,2 | 53,8 ± 5,0 | |
| **S37** | **Y-H-S-Bpa-E-Y-***C* | 39,1 ± 1,2 | | |
| **S38** | **Y-H-S-W-E,-Bpa-***C* | 52,3 ± 4,1 | 59,2 ± 4,8 | |
| **S39** | **Y-H-Abu-W-D-Y-***C* | 46,4 ± 0,8 | | |
| **S40** | **Y-C-S-W-E-Phg** | 38,2 ± 3,4 | 54,9 ± 4,0 | |
| **S40** | **Y-S-W-E-Y-***C* | 50,2 ± 0,6 | | |
| **S42** | **Y-H-S-(2-Nal)-E-Y-***C* | 60,3 ± 2,2 | 47,8 ± 2,1 | |
| **S43** | **S-W-D-Y-***C* | 48,2 ± 2,8 | 35,6 ± 2,6 | |
| **S44** | **E-S-W-E-Y-***C* | 38,0 ± 2,4 | 35,1 ± 2,4 | |
| **S45** | **Ac-E-S-W-E-Y-***C* | 36,4 ± 2,6 | 38,4 ± 2,6 | |
| **S46** | **T-W-E-Y-***C* | 37,0 ± 2,1 | 25,1 ± 1,9 | |
| **S47** | **Y-H-T-W-E-Y-***C* | 47,5 ± 2,9 | 38,9 ± 2,8 | |
| **S48** | **Y-H-S-W-D-Y-***C* | 52,0 ± 3,1 | 40,2 ± 2,9 | |
| **S49** | **Y-F-Abu-W-E-Y-***C* | 64,5 ± 4,0 | 54,4 ± 3,8 | |
| **S50** | **Abu-W-E-Y-***C* | 35,3 ± 2,4 | 36,4 ± 2,4 | |
| **S51** | **Y-H-S-Bpa-E-Bpa-***C* | 63,4 ± 3,8 | 49,3 ±3,5 | |
| **S52** | **Y-H-T-W-D-Y-***C* | 47,5 ± 3,3 | 51,2 ± 3,4 | |
| **S53** | **S-W-E-Phg-***C* | 38,1 ±2,7 | 42,5 ± 2,8 | |
| S54 | **Y-H-Abu-W-E-Y-***C* | 32,3 ± 2,6 | 44,4 ± 2,8 | |
| S55 | **Y-H-S-W-E-Bip(4,4')-***C* | 51,5 ± 2,9 | 36,2 ± 2,1 | |
| S56 | **Y-H-S-W-E-(2-Nal)-***C* | 51,5 ± 3,4 | 51,5 ± 3,4 | |
| S57 | **S-Bpa-E-(1-Nal)-***C* | 35,4 ± 6,6 | | |
| S58 | **S-Bpa-E-(2-Nal)-***C* | 42,0 ± 5,3 | | |
| S59 | **S-Bpa-E-Bip(4,4')-***C* | 30,9 ± 2,1 | | |
| S60 | **S-Bpa-E-Phe(4-Cl)-***C* | 33,9 ± 3,2 | | |
| S61 | **S-Bpa-E-Bpa-***C* | 11,1 ± 3,3 | | |
| S62 | **S-(1-Nal)-E-Bip(4,4')-***C* | 38,6 ± 0,4 | | |
| S63 | **S-(2-Nal)-E-Bip(4,4')-***C* | 41,4 ± 1,2 | | |
| S64 | **S-Bip(4,4')-E-Bpa-***C* | 36,2 ± 1,5 | | |
| S65 | **S-Bta-E-Bpa-***C* | 39,2 ± 2,4 | | |
| S66 | **S-Phe(4-Cl)-E-Bpa-***C* | 40,0 ± 2,5 | | |
| S67 | **S-Phe(3,4-Cl)-E-Bpa-***C* | 47,7 ± 0,0 | | |
| S68 | **Y-Y-S-W-E-Y-***C* | 54,3 ± 2,1 | | |
| S69 | **(*N*-Me)Y-C-S-W-E-Y** | 7,1 ± 0,7 | | 10,3 |
| S70 | **Y-C-S-(*N*-Me)W-E-Y** | 72,2 ±1,7 | | 13,8 |
| S71 | **S-Bpa-E-Y-***C* | 30,6 ± 0,4 | | |
| S72 | **S-Bpa-E-Phg-***C* | 41,3 ± 1,4 | | |
| S73 | **S-W-E-Bpa-***C* | 40,0 ± 2,8 | | |
| S74 | **Y-H-S-W-E-Phg-***C* | 34,9 ± 0,4 | | |
| S75 | **Y-H-S-Bpa-E-Phg-***C* | 28,1 ± 0,3 | | |
| S76 | **Y-H-V-W-E-Y-***C* | 26,6 ± 1,3 | | |
| S77 | **Y-H-S-Phe(3,4-Cl)-E-Y-***C* | 34,4 ± 3,2 | | |
| S78 | **Y-H-S-Bta-E-Y-***C* | 6,0 ± 0,8 | | |
| S79 | **Y-W-E-Y-***C* | 47,7 ± 0,45 | | |

The compounds of formula I can be understood as peptides, non-natural peptides or peptide derivatives and may be partially or completely synthesized, for example using solution or solid state synthesis techniques known in the art (Gysin, B. F.; Merrifield, R. B. J. Am. Chem. Soc. 1972, 94, 3102; or Merrifield, R. B. Angew. Chemie Int. Ed. 1985, 24(10), 799-810) applying appropriate amino or carboxy building blocks. A sequential synthesis is contemplated. Other organic synthetic methods may be employed in the synthesis of the compounds according to formula I, such as the methods described in the textbook Houben-Weyl Methods in Organic Chemistry, Vol. 22 a - d, Editor-in-Chief: M. Goodman, Thieme Verlag, Stuttgart, 2003.

If desired, the starting materials can also be formed *in situ* without isolating them from the reaction mixture, but instead subsequently converting them further into the compounds of the formula I.

Suitable inert solvents are, for example, hydrocarbons, such as hexane, petroleum ether, benzene, toluene, or xylene; chlorinated hydrocarbons, such as trichlorethylene, 1,2-dichloroethane, tetrachloromethane, chloroform or dichloromethane; alcohols, such as methanol, ethanol, isopropanol, n-propanol, *n*-butanol or *tert*-butanol; ethers, such as diethyl ether, diisopropyl ether, tetrahydrofurane (THF) or dioxane; glycol ethers, such as 1,2-dimethoxyethane, acetamide, such as *N*-methylpyrrolidone, dimethylacetamide or dimethylformamide (DMF); nitriles, such as acetonitrile; sulfoxides, such as dimethyl sulfoxide (DMSO); carbon disulfide; carboxylic acids, such as formic acid or acetic acid; nitro compounds, such as nitromethane or nitrobenzene; esters, such as ethyl acetate, water, or mixtures of the said solvents.

The compounds of the formula I can furthermore be obtained by liberation from a functional derivative by solvolysis, such as hydrolysis, or hydrogenolysis.

Preferred starting materials for the solvolysis or hydrogenolysis are those having corresponding protected amino and/or hydroxyl groups instead of one or more free amino and/or hydroxyl groups, preferably those which carry an amino-protecting group instead of an H atom bonded to an N atom, for example those which conform to the formula I, but contain an NHR' group (in which R' is an amino-protecting group, for example BOC or CBZ) instead of an NH₂ group.

Preference is furthermore given to starting materials which carry a hydroxyl-protecting group instead of the H atom of a hydroxyl group, for example those which conform to the formula I, but contain an R"O-phenyl group (in which R" is a hydroxyl-protecting group, for example tert-butyl or benzyl) instead of a hydroxy-phenyl group. In other words, the hydroxyl group covalently bonded to the aromatic ring is protected from transformation by a protecting group.

Preference is furthermore given to starting materials which carry a carboxyl-protecting group instead of the H atom of a carboxyl group, for example those which conform to the formula I, but contain an R"'O-CO- group (in which R"' is a carboxyl-protecting group, for example tert-butyl or benzyl) instead of a carboxyl group. In other words, the oxygen atom of the carboxyl group is protected from transformation by a protecting group.

It is also possible for more than one - identical or different - protecting group to be present in the molecule. If the more than one protecting groups present are different from one another, this offers an advantage in that they can be cleaved off selectively.

The term "amino-protecting group" is known in general terms and relates to groups which are suitable for protecting (blocking) an amino group against chemical reactions, but are easy to remove. Typical for such groups are, in particular, unsubstituted or substituted acyl, aryl, aralkoxymethyl or aralkyl groups. Because the amino-protecting groups are removed after the desired reaction (or reaction sequence) occurs, their type and size are furthermore not crucial; however, preference is given to those having 1-20, in particular 1-8, carbon atoms. The term "acyl group" includes acyl groups derived from aliphatic, araliphatic, aromatic or heterocyclic carboxylic acids or sulfonic acids, and, in particular, alkoxycarbonyl, aryloxycarbonyl and especially aralkoxycarbonyl groups. Examples of such acyl groups are alkanoyl, such as acetyl, propionyl, butyryl; aralkanoyl such as phenylacetyl; aroyl such as benzoyl oder toluyl; aryloxyalkanoyl such as POA; alkoxycarbonyl such as methoxycarbonyl, ethoxycarbonyl, 2,2,2-trichlorethoxycarbonyl, BOC, 2-iodethoxycarbonyl; aralkyloxycarbonyl such as CBZ ("carbobenzoxy"), 4-methoxybenzyloxycarbonyl, Fmoc; arylsulfonyl such as Mtr, Pbf or Pmc. Preferred amino-protecting groups are BOC, Mtr, CBZ, Fmoc, Benzyl and Acetyl groups.

The term "hydroxyl-protecting group" is likewise known in general terms and relates to groups which are suitable for protecting a hydroxyl group against chemical reactions, but are easy to remove after the desired chemical reaction has been carried out elsewhere in the molecule. Typical for such groups are the above-mentioned unsubstituted or substituted aryl, aralkyl or acyl groups, furthermore also alkyl groups. The nature and size of the hydroxyl-protecting groups are not crucial since they are removed again after the desired chemical reaction or reaction sequence; preference is given to groups having 1-20, in particular 1-10, carbon atoms. Examples of hydroxyl-protecting groups are, inter alia, benzyl, p-nitrobenzoyl, *tert*-butyl and acetyl, where benzyl and *tert*-butyl are particularly preferred.

The term "carboxyl-protecting group" is likewise known in general terms and relates to groups which are suitable for protecting a carboxyl group against chemical reactions, but are easy to remove after the desired chemical reaction has been carried out elsewhere in the molecule. Typical for such groups are the above-mentioned unsubstituted or substituted aryl, aralkyl or acyl groups, furthermore also alkyl groups. The nature and size of the hydroxyl-protecting groups are not crucial since they are removed again after the desired chemical reaction or reaction sequence; preference is given to groups having 1-20, in particular 1-10, carbon atoms. Examples of carboxyl-protecting groups are, inter alia, benzyl, *tert*-butyl and acetyl, where benzyl and tert-butyl are particularly preferred.

The compounds of the formula I are liberated from their functional derivatives - depending on the protecting group used - for example using strong acids, advantageously using TFA or perchloric acid, but also strong inorganic acids, such as hydrochloric acid or sulfuric acid, strong organic carboxylic acids, such as trichloroacetic acid, or sulfonic acids, such as benzenesulfonic acid or p-toluenesulfonic acid. The presence of an additional inert solvent is possible, but is not always necessary. Suitable inert solvents are preferably organic, for example carboxylic acids, such as acetic acid, ethers, such as tetrahydrofurane or dioxane, amides, such as DMF, halogenated hydrocarbons, such as dichloromethane, furthermore also alcohols, such as methanol, ethanol or isopropanol, and water. Mixtures of above-mentioned solvents are furthermore suitable. TFA is preferably used in excess without addition of a further solvent, and perchloric acid is preferably used in the form of a mixture of acetic acid and 70% perchloric acid in the ratio 9:1. The reaction temperatures for the cleavage are advantageously between about 0° and about 50°, preferably between 15° and 30° (room temperature).

The BOC, Obut, Pbf, Pmc and Mtr groups can, for example, preferably be cleaved off using TFA in dichloromethane or using approximately 3 to 5N HCl in dioxane at 15-30°, and the Fmoc group can be cleaved off using an approximately 5 to 50% solution of dimethylamine, diethylamine or piperidine in DMF at 15-30°.

The trityl group is employed to protect the amino acids histidine, asparagine, glutamine and cysteine. They are cleaved off using TFA / 10% thiophenol, TFA / anisole, TFA / thioanisole or TFA/TIPS/H2O, with the trityl group being cleaved off all the said amino acids.

The Pbf (pentamethylbenzofuranyl) group is employed to protect Arg. It is cleaved off using, for example TFA in dichloromethane.

Hydrogenolytically removable protecting groups (for example CBZ or benzyl) can be cleaved off, for example, by treatment with hydrogen in the presence of a catalyst (for example a noble-metal catalyst, such as palladium, advantageously on a solid carrier, such as carbon). Suitable solvents here are those indicated above, in particular, for example, alcohols, such as methanol or ethanol, or amides, such as DMF. The hydrogenolysis is generally carried out at temperatures between 0° and 100° and pressures between 1 and 200 bar, preferably at 10-30° and 1-10 bar. Hydrogenolysis of the CBZ group succeeds well, for example, on 5 to 10% Pd/C in methanol or using ammonium formate (instead of hydrogen) on Pd/C in methanol/DMF at 10-30°.

A base of the formula I can be converted into the associated acid-addition salt using an acid, for example by reaction of equivalent amounts of the base and the acid in an inert solvent, such as ethanol, followed by evaporation. Thus it is possible to use inorganic acids, for example sulfuric acid, nitric acid, a hydrohalic acid, such as hydrochloric acid or hydrobromic acid, phosphoric acids, such as orthophosphoric acid or sulfamic acid. Organic acids may be employed including aliphatic, alicyclic, araliphatic, aromatic or heteroaromatic monobasic or polybasic carboxylic, sulfonic or sulfuric acids, for example formic acid, acetic acid, triflouroacetic acid, propionic acid, pivalic acid, diethylacetic acid, malonic acid, succinic acid, pimelic acid, fumaric acid, malenic acid, lacitic acid, tartaric acid, malic acid, citric acid, gluconic acid, ascorbic acid, nicotinic acid, isonicotinic acid, methane- or ethanesulfonic acid, ethanedisulfonic acid, 2-hydroxyethanesulfonic acid, benzenesulfonic acid, p-toluenesulfonic acid, naphthalenemono- and -disulfonic acids and laurylsulfuric acid. Salts, for example picrates, can also be used for the isolation and/or purification of the compounds of the formula I.

On the other hand, an acid of the formula I can be converted into one of its metal of ammonium salts by reaction with a base. Suitable salts here are, in particular, the sodium, potassium, magnesium, calcium and ammonium salts, furthermore substituted ammonium salts, for example the dimethyl-, diethyl- or diisopropyl-ammonium salts, monoethanol-diethanol- or diisopropanolylammonium salts, cyclohexyl-, dicyclohexylammonium salts, dibenzylethylenediammonium salts, furthermore, for example, salts with arginine or lysine.

Chromatographic methods were used according to the following parameters.
RT = retention time (minutes) on HPLC in the following system:

| | |
|---|---|
| Column: | YMC ODS A RP 5C₁₈, 250 x 4,6 mm |
| Eluent A: | 0.1 % TFA in water |
| Eluent B: | 0.1 % TFA in acetonitrile |
| Flow rate: | 1 mL/min |
| Gradient: | 10 - 50 % B / 30 min. |
| | |
| Mass spectrometry (MS): ESI (electrospray ionisation) (M + H)⁺ | |

Rink-amid resin stands for 4-(2',4'-dimethoxyphenyl-Fmoc-aminomethyl)-phenoxy resin, which allows, for example, the synthesis of peptides and peptido mimetic derivatives with C-terminal -CONH₂ groups, TCP resin denotes trityl chloride-polystyrene resin.

### Example 1: Preparation of compounds as affinity ligands for FVIII and binding of pd-FVIII

Peptides **S1** to **S79** were immobilized on the Toyopearl AF-Epoxy-650M resin (Tosoh Biosep) as described by Jungbauer *et al.* For immobilization, 2.5 mg of each peptide was dissolved in 0.25 mL of the immobilization buffer (0.2 M sodium bicarbonate, pH 10.3), and 0.036 g of the dry resin powder (corresponding to 0.125 mL of swollen resin) was added, followed by incubation of the mixture with gentle rotation for 48 hours. Upon incubation for 48 hours the resin was washed once with immobilization buffer, once with 1 M NaCl and then 3 times with binding buffer, and binding of ¹²⁵I-labeled pd-FVIII to the peptide-coated and control resin was tested. The coupling density of each peptide in each of the reported experiments was as mentioned in Table 1. The control 0.25 mL portion of the resin was similarly treated in parallel experiment in the absence of peptide and was subsequently used as a control (designated as Background) in ¹²⁵I-pd-FVIII binding experiments. ¹²⁵I-pd-FVIII Bound/Background ratios were calculated as the amount of ¹²⁵I-pd-FVIII, bound to an immobilized peptide, divided by that bound to uncoated control resin, prepared as described above. This ratio represents a Signal/Noise ratio for the micro-beads assay, since ¹²⁵I-pd-FVIII bound to peptide represents the signal value and ¹²⁵I-pd-FVIII bound to peptide-uncoated resin represents the background (noise) value.

Plasma-derived (pd-) human Factor VIII (FVIII) was purified from concentrate by immunoaffinity chromatography on an anti-FVIII monoclonal antibody column followed by subsequent concentration of pd-FVIII by ion-exchange chromatography using Resource Q HR5/5 column. To separate FVIII from vWf, concentrate was incubated in 0.35 M NaCl, 0.04 M CaCl₂, prior to affinity purification.

The resin with immobilized peptides was washed in the binding buffer (0.01 M Hepes, 0.1 M NaCl, 5 mM CaCl₂, 0.01% Tween-80). Subsequently, the resin was diluted in the binding buffer as 1:7 slurry and aliquoted into Eppendorf tubes (40 µL per tube). ¹²⁵I-pd-FVIII (100000 cpm in 10 µL) was added to the tubes and the volume of the mixture was adjusted to 100 µL by adding 50 µL of the binding buffer containing 4% BSA to give a 2% final concentration of BSA. After 2 hours of incubation at room temperature on a rotator, the samples were washed 4 times in the binding. After each wash the tubes were centrifuged at 5000 rpm for 1 min in an Eppendorf microcentrifuge, supernatant was discarded, and the resin was re-suspended in the washing buffer, followed by centrifugation under the same conditions. After four washes the tubes with pellet without supernatant were counted for radioactivity. The resin without peptide was processed similarly to account for non-specific pd-FVIII binding to tubes and resin itself, and the radioactivity in this control tube was considered as a background value. Since ¹²⁵I-pd-FVIII contains the some fraction damaged during radiolabeling, binding was calculated as a percent of maximal achievable binding, determined in separate experiment with anti-FVIII Mab 8860 -coated resin.

All the measurements were performed in duplicates. Each experiment was performed using two independently prepared immobilized peptide samples. The data presented in each figure are the mean values of the four determinations: duplicate determination in the two assays performed with beads on which peptides were independently immobilized on the different days. The value of standard deviation of the above quadruplicate determinations (duplicate determinations in two independent experiments) were typically less than 10% of the measured values of ¹²⁵I-pd-FVIII binding to peptides.

The compounds **S1** to **S79** can be synthesized via solid phase peptide synthesis using Fmoc-strategy on TCP resin and on Rink-amide resin respectively (see Fields, G. B.; Nobie, R. L. Int. J. Pept. Protein Res. 1990, 35, 161).

The cleavage of the peptides derivatives from the solid phase and the cleavage of their side chain protection groups can be done simultaneously using 90% TFA, 5% H₂O and 5% TIPS.

All compounds were purified by preparative HPLC.

### Example 2: Development of novel small peptides (tetrapeptides to hexapeptide) with high affinity to FVIII. Structures comprising C-terminal cysteine as linker molecule

Pflegerl et al. (J. Peptide Res. 2002, 59, 174-182) reported the development of different octapeptides (e.g. EYHSWEYC) with high affinity towards FVIII. The essential amino acid sequence was found to be WEY, located in the C-terminal side of the peptides. Minimizing and optimizing the peptide sequence led the inventors to the development of a series of FVIII binding molecules which proved to bind Factor VIII with high affinity. These novel compounds comprise of tetrapeptides, pentapeptides, hexapeptides, and heptapeptides (According to the present invention, cysteine is not among the binding sequence. Therefore: e.g. HSWEYC is seen as a pentapeptide HSWEY with a cysteine attached as linker) with a C-terminal attached liker group (cysteine) like the following, given as examples in Table 2:

**Table 2**

| **Sequence** | **% Binding Factor VIII** | |
|---|---|---|
| **Novel compounds** | | |
| **HSWEY*C*** | 54 | **S29** |
| **Ac-SWEY*C*** | 43 | **S30** |
| **Ac-HSWEY*C*** | 49 | **S31** |
| **Ac-YHSWEY***C* | 62 | **S32** |
| **YFAbuWEF***C* | 53 | **S33** |
| **YfSWEY*C*** | 54 | **S34** |

| *Reference* | | |
|---|---|---|
| **EYHSWEYC** | 50 | |

| | | |
|---|---|---|
| *The binding molecule is marked in bold, the linker group is marked in italics. Abu: α-aminobutyric acid* | | |

These minimized compounds show an even improved FVIII binding affinity related to the lead octapeptide EYHSWEYC. Like the peptides mentioned in WO 99/14232 und US 2003-165822 the present novel peptides were immobilized on a matrix (here Toyopearl AF-Epoxy-650M resin) via a C-terminal linker group.

### Example 3: Development of novel hexapeptides with high affinity to FVIII. Structures comprising the linker molecule cysteine within the binding sequence

On the basis of the lead octapeptide EYHSWEYC the present inventors were able to develop a novel class of hexapeptides with particularly high binding affinity to FVIII:

The N-terminal E1 and the C-terminal C8 were deleted and the linker cysteine introduced within the binding sequence by substituting H3 with C. This modification led to the minimized hexapeptide S7 with greatly improved FVIII binding affinity. Such structures, with the linker located within the binding sequence, have not been described in the literature before. Additional sequence optimizations of **S7** resulted in further remarkable improvements of the FVIII binding ability (e.g. **S9, S10, S11**). These novel peptides even proved to bind FVIII with a much higher efficiency than the small peptides and peptido-mimetics described in a previous provisional patent application of the present inventors, see Table 3:

**Table 3**

| | **Sequence** | **% Binding Factor VIII** |
|---|---|---|
| **S7** | **YCSWEY** | 64% |
| **S9** | **Ac-YCSWEY** | 82% |
| **S16** | **YCAWDY** | 72% |
| **S18** | **YCVWDY** | 72% |
| **S10** | **YCTWEY** | 80% |
| **S11** | **YCSWDY** | 80% |
| **S12** | **YCTWDY** | 74% |
| **S8** | **yewscy** | 69% |
| **S17** | **y d w a C y** | 75% |
| **S19** | **y d w v C y** | 73% |

| | | |
|---|---|---|
| *The binding molecule is marked in bold, the linker group is marked in italics and small letters represent D-amino acids.* | | |

Novel compounds are furthermore the retro-inverso peptides **S8, S11** and **S19** which have been derived from the peptides **S7, S16** and **S18.**

In general, retro-inverso peptides are synthesized with a reversed sequence, concurrently the configuration of every amino acid is inversed.

Comparing the corresponding structures **(S7** and **S8, S16** and **S17, S18** and **S19)** the position and direction of the single amino acid side chains are conserved while the direction of the peptide bonds are inversed and the C- and the N-terminus are replaced by each other.

The resulting compounds **S8, S17 and S19** do not contain any proteinogenic amino acid and provide a particularly high protease stability which is crucial for purifying FVIII directly out of protease containing media such as serum or cell culture supernatants. The use of retro-inverso peptides as affinity ligands for FVIII has not been described in the literature before.

SEQUENCE LISTING

<110> Technische Universität München

<120> Minimized small peptides with high affinity for Factor VIII and
      Factor VIII-like Proteins

<130> 118416 b/mdj

<140> EP 06013852.6
<141> 2006-07-04

<160> 79

<170> PatentIn version 3.4

<210> 1
<211> 6
<212> PRT
<213> Artificial

<220>
<223> Synthetic peptide with high affinity for Factor VIII and Factor
      VIII-like Proteins


<220>
<221> MOD_RES
<222> (6)..(6)
<223> AMIDATION

<400> 1

Tyr Cys Ser Trp Glu Tyr
1               5


<210> 2
<211> 6
<212> PRT
<213> Artificial

<220>
<223> Synthetic peptide with high affinity for Factor VIII and Factor
      VIII-like Proteins


<220>
<221> MOD_RES
<222> (1)..(1)
<223> ACETYLATION

<220>
<221> MOD_RES
<222> (6)..(6)
<223> AMIDATION

<400> 2

Tyr Cys Ser Trp Glu Tyr
1               5


<210> 3
<211> 6
<212> PRT
<213> Artificial

<220>

<223>  Synthetic peptide with high affinity for Factor VIII and Factor
       VIII-like Proteins

<220>
<221>  MOD_RES
<222>  (6)..(6)
<223>  AMIDATION

<400>  3

Tyr Cys Thr Trp Asp Tyr
1               5

<210>  4
<211>  6
<212>  PRT
<213>  Artificial

<220>
<223>  Synthetic peptide with high affinity for Factor VIII and Factor
       VIII-like Proteins

<220>
<221>  MOD_RES
<222>  (1)..(1)
<223>  ACETYLATION

<220>
<221>  MOD_RES
<222>  (6)..(6)
<223>  AMIDATION

<400>  4

Tyr Cys Thr Trp Asp Tyr
1               5

<210>  5
<211>  6
<212>  PRT
<213>  Artificial

<220>
<223>  Synthetic peptide with high affinity for Factor VIII and Factor
       VIII-like Proteins

<220>
<221>  MISC_FEATURE
<222>  (2)..(2)
<223>  L-HomoCys, L-Homocysteine

<400>  5

Tyr Cys Ser Trp Glu Tyr
1               5

<210>  6
<211>  6
<212>  PRT
<213>  Artificial

<220>
<223>  Synthetic peptide with high affinity for Factor VIII and Factor

VIII-like Proteins

<400> 6

000


<210> 7
<211> 6
<212> PRT
<213> Artificial

<220>
<223> Synthetic peptide with high affinity for Factor VIII and Factor
      VIII-like Proteins

<400> 7

Tyr Cys Ser Trp Glu Tyr
1               5


<210> 8
<211> 6
<212> PRT
<213> Artificial

<220>
<223> Synthetic peptide with high affinity for Factor VIII and Factor
      VIII-like Proteins

<400> 8

000


<210> 9
<211> 7
<212> PRT
<213> Artificial

<220>
<223> Synthetic peptide with high affinity for Factor VIII and Factor
      VIII-like Proteins


<220>
<221> MOD_RES
<222> (1)..(1)
<223> ACETYLATION

<400> 9

Tyr Tyr Cys Ser Trp Glu Tyr
1               5


<210> 10
<211> 6
<212> PRT
<213> Artificial

<220>
<223> Synthetic peptide with high affinity for Factor VIII and Factor
      VIII-like Proteins

<400> 10

Tyr Cys Thr Trp Glu Tyr

1                5


<210>  11
<211>  6
<212>  PRT
<213>  Artificial

<220>
<223>  Synthetic peptide with high affinity for Factor VIII and Factor
       VIII-like Proteins

<400>  11

Tyr Cys Ser Trp Asp Tyr
1               5


<210>  12
<211>  6
<212>  PRT
<213>  Artificial

<220>
<223>  Synthetic peptide with high affinity for Factor VIII and Factor
       VIII-like Proteins

<400>  12

Tyr Cys Thr Trp Asp Tyr
1               5


<210>  13
<211>  6
<212>  PRT
<213>  Artificial

<220>
<223>  Synthetic peptide with high affinity for Factor VIII and Factor
       VIII-like Proteins

<400>  13

000

<210>  14
<211>  6
<212>  PRT
<213>  Artificial

<220>
<223>  Synthetic peptide with high affinity for Factor VIII and Factor
       VIII-like Proteins


<220>
<221>  MOD_RES
<222>  (1)..(1)
<223>  ACETYLATION

<400>  14

Tyr Cys Thr Trp Asp Tyr
1               5


<210>  15
<211>  6

<212>  PRT
<213>  Artificial

<220>
<223>  Synthetic peptide with high affinity for Factor VIII and Factor
       VIII-like Proteins


<220>
<221>  MISC_FEATURE
<222>  (4)..(4)
<223>  p-Benzoyl-L-Phe, p-Benzoyl-L-Phenylalanine

<400>  15

Tyr Cys Ser Phe Glu Tyr
1               5


<210>  16
<211>  6
<212>  PRT
<213>  Artificial

<220>
<223>  Synthetic peptide with high affinity for Factor VIII and Factor
       VIII-like Proteins

<400>  16

Tyr Cys Ala Trp Asp Tyr
1               5


<210>  17
<211>  6
<212>  PRT
<213>  Artificial

<220>
<223>  Synthetic peptide with high affinity for Factor VIII and Factor
       VIII-like Proteins

<400>  17

000


<210>  18
<211>  6
<212>  PRT
<213>  Artificial

<220>
<223>  Synthetic peptide with high affinity for Factor VIII and Factor
       VIII-like Proteins

<400>  18

Tyr Cys Val Trp Asp Tyr
1               5


<210>  19
<211>  6
<212>  PRT
<213>  Artificial

<220>

<223>   Synthetic peptide with high affinity for Factor VIII and Factor
        VIII-like Proteins

<400>   19

000


<210>   20
<211>   5
<212>   PRT
<213>   Artificial

<220>
<223>   Synthetic peptide with high affinity for Factor VIII and Factor
        VIII-like Proteins

<400>   20

Cys Ser Trp Glu Tyr
1               5


<210>   21
<211>   5
<212>   PRT
<213>   Artificial

<220>
<223>   Synthetic peptide with high affinity for Factor VIII and Factor
        VIII-like Proteins


<220>
<221>   MOD_RES
<222>   (1)..(1)
<223>   METHYLATION

<400>   21

Ser Trp Glu Tyr Cys
1               5


<210>   22
<211>   7
<212>   PRT
<213>   Artificial

<220>
<223>   Synthetic peptide with high affinity for Factor VIII and Factor
        VIII-like Proteins


<220>
<221>   MOD_RES
<222>   (3)..(3)
<223>   METHYLATION

<400>   22

Tyr Phe Ser Trp Glu Tyr Cys
1               5


<210>   23
<211>   7
<212>   PRT
<213>   Artificial

<220>
<223>  Synthetic peptide with high affinity for Factor VIII and Factor
      VIII-like Proteins

<220>
<221>  MOD_RES
<222>  (5)..(5)
<223>  METHYLATION

<400>  23

Tyr Phe Ser Trp Glu Tyr Cys
1               5

<210>  24
<211>  6
<212>  PRT
<213>  Artificial

<220>
<223>  Synthetic peptide with high affinity for Factor VIII and Factor
      VIII-like Proteins

<220>
<221>  MOD_RES
<222>  (3)..(3)
<223>  METHYLATION

<400>  24

Tyr Cys Glu Trp Glu Tyr
1               5

<210>  25
<211>  6
<212>  PRT
<213>  Artificial

<220>
<223>  Synthetic peptide with high affinity for Factor VIII and Factor
      VIII-like Proteins

<220>
<221>  MOD_RES
<222>  (5)..(5)
<223>  METHYLATION

<400>  25

Tyr Cys Ser Trp Glu Tyr
1               5

<210>  26
<211>  6
<212>  PRT
<213>  Artificial

<220>
<223>  Synthetic peptide with high affinity for Factor VIII and Factor
      VIII-like Proteins

<220>
<221>  MOD_RES
<222>  (6)..(6)
<223>  METHYLATION

<400>  26

Tyr Cys Ser Trp Glu Tyr
1               5


<210>  27
<211>  7
<212>  PRT
<213>  Artificial

<220>
<223>  Synthetic peptide with high affinity for Factor VIII and Factor
       VIII-like Proteins

<400>  27

000


<210>  28
<211>  7
<212>  PRT
<213>  Artificial

<220>
<223>  Synthetic peptide with high affinity for Factor VIII and Factor
       VIII-like Proteins

<400>  28

000


<210>  29
<211>  6
<212>  PRT
<213>  Artificial

<220>
<223>  Synthetic peptide with high affinity for Factor VIII and Factor
       VIII-like Proteins

<400>  29

His Ser Trp Glu Tyr Cys
1               5


<210>  30
<211>  5
<212>  PRT
<213>  Artificial

<220>
<223>  Synthetic peptide with high affinity for Factor VIII and Factor
       VIII-like Proteins


<220>
<221>  MOD_RES
<222>  (1)..(1)
<223>  ACETYLATION

<400>  30

Ser Trp Glu Tyr Cys
1               5


<210>  31
<211>  6
<212>  PRT
<213>  Artificial

<220>
<223>  Synthetic peptide with high affinity for Factor VIII and Factor
       VIII-like Proteins


<220>
<221>  MOD_RES
<222>  (1)..(1)
<223>  ACETYLATION

<400>  31

His Ser Trp Glu Tyr Cys
1               5


<210>  32
<211>  7
<212>  PRT
<213>  Artificial

<220>
<223>  Synthetic peptide with high affinity for Factor VIII and Factor
       VIII-like Proteins


<220>
<221>  MOD_RES
<222>  (1)..(1)
<223>  ACETYLATION

<400>  32

Tyr His Ser Trp Glu Tyr Cys
1               5


<210>  33
<211>  7
<212>  PRT
<213>  Artificial

<220>
<223>  Synthetic peptide with high affinity for Factor VIII and Factor
       VIII-like Proteins


<220>
<221>  MOD_RES
<222>  (3)..(3)
<223>  4Abu

<400>  33

Tyr Phe Xaa Trp Glu Phe Cys
1               5

<210>  34
<211>  7
<212>  PRT
<213>  Artificial

<220>
<223>  Synthetic peptide with high affinity for Factor VIII and Factor
       VIII-like Proteins

<400>  34

000


<210>  35
<211>  5
<212>  PRT
<213>  Artificial

<220>
<223>  Synthetic peptide with high affinity for Factor VIII and Factor
       VIII-like Proteins

<400>  35

000


<210>  36
<211>  7
<212>  PRT
<213>  Artificial

<220>
<223>  Synthetic peptide with high affinity for Factor VIII and Factor
       VIII-like Proteins


<220>
<221>  MOD_RES
<222>  (3)..(3)
<223>  4Abu

<400>  36

Tyr Phe Xaa Trp Glu Tyr Cys
1               5


<210>  37
<211>  7
<212>  PRT
<213>  Artificial

<220>
<223>  Synthetic peptide with high affinity for Factor VIII and Factor
       VIII-like Proteins


<220>
<221>  MISC_FEATURE
<222>  (4)..(4)
<223>  p-Benzoyl-L-Phe, p-Benzoyl-L-Phenylalanine

<400>  37

Tyr His Ser Phe Glu Tyr Cys
1               5

<210>  38
<211>  7
<212>  PRT
<213>  Artificial

<220>
<223>  Synthetic peptide with high affinity for Factor VIII and Factor
       VIII-like Proteins


<220>
<221>  MISC_FEATURE
<222>  (6)..(6)
<223>  p-Benzoyl-L-Phe, p-Benzoyl-L-Phenylalanine

<400>  38

Tyr His Ser Trp Glu Phe Cys
1               5


<210>  39
<211>  7
<212>  PRT
<213>  Artificial

<220>
<223>  Synthetic peptide with high affinity for Factor VIII and Factor
       VIII-like Proteins


<220>
<221>  MOD_RES
<222>  (3)..(3)
<223>  4Abu

<400>  39

Tyr His Xaa Trp Asp Tyr Cys
1               5


<210>  40
<211>  6
<212>  PRT
<213>  Artificial

<220>
<223>  Synthetic peptide with high affinity for Factor VIII and Factor
       VIII-like Proteins


<220>
<221>  MISC_FEATURE
<222>  (6)..(6)
<223>  L-Phg, L-Phenylglycine

<400>  40

Tyr Cys Ser Trp Glu Xaa
1               5


<210>  41
<211>  6
<212>  PRT
<213>  Artificial

<220>
<223>  Synthetic peptide with high affinity for Factor VIII and Factor
       VIII-like Proteins

<400>  41

Tyr Ser Trp Glu Tyr Cys
1               5


<210>  42
<211>  7
<212>  PRT
<213>  Artificial

<220>
<223>  Synthetic peptide with high affinity for Factor VIII and Factor
       VIII-like Proteins


<220>
<221>  MISC_FEATURE
<222>  (4)..(4)
<223>  L-2-Nal, L-2-Naphthylalanine

<400>  42

Tyr His Ser Xaa Glu Tyr Cys
1               5


<210>  43
<211>  5
<212>  PRT
<213>  Artificial

<220>
<223>  Synthetic peptide with high affinity for Factor VIII and Factor
       VIII-like Proteins

<400>  43

Ser Trp Asp Tyr Cys
1               5


<210>  44
<211>  6
<212>  PRT
<213>  Artificial

<220>
<223>  Synthetic peptide with high affinity for Factor VIII and Factor
       VIII-like Proteins

<400>  44

Glu Ser Trp Glu Tyr Cys
1               5


<210>  45
<211>  6
<212>  PRT
<213>  Artificial

<220>
<223>  Synthetic peptide with high affinity for Factor VIII and Factor
       VIII-like Proteins

<220>
<221>  MOD_RES
<222>  (1)..(1)
<223>  ACETYLATION

<400>  45

Glu Ser Trp Glu Tyr Cys
1               5


<210>  46
<211>  5
<212>  PRT
<213>  Artificial

<220>
<223>  Synthetic peptide with high affinity for Factor VIII and Factor
       VIII-like Proteins

<400>  46

Thr Trp Glu Tyr Cys
1               5


<210>  47
<211>  7
<212>  PRT
<213>  Artificial

<220>
<223>  Synthetic peptide with high affinity for Factor VIII and Factor
       VIII-like Proteins

<400>  47

Tyr His Thr Trp Glu Tyr Cys
1               5


<210>  48
<211>  7
<212>  PRT
<213>  Artificial

<220>
<223>  Synthetic peptide with high affinity for Factor VIII and Factor
       VIII-like Proteins

<400>  48

Tyr His Ser Trp Asp Tyr Cys
1               5


<210>  49
<211>  7
<212>  PRT
<213>  Artificial

<220>
<223>  Synthetic peptide with high affinity for Factor VIII and Factor
       VIII-like Proteins


<220>

<221>  MOD_RES
<222>  (3)..(3)
<223>  4Abu

<400>  49

Tyr Phe Xaa Trp Glu Tyr Cys
1               5


<210>  50
<211>  5
<212>  PRT
<213>  Artificial

<220>
<223>  Synthetic peptide with high affinity for Factor VIII and Factor
       VIII-like Proteins


<220>
<221>  MOD_RES
<222>  (1)..(1)
<223>  4Abu

<400>  50

Xaa Trp Glu Tyr Cys
1               5


<210>  51
<211>  7
<212>  PRT
<213>  Artificial

<220>
<223>  Synthetic peptide with high affinity for Factor VIII and Factor
       VIII-like Proteins


<220>
<221>  MISC_FEATURE
<222>  (4)..(4)
<223>  p-Benzoyl-L-Phe, p-Benzoyl-L-Phenylalanine

<220>
<221>  MISC_FEATURE
<222>  (6)..(6)
<223>  p-Benzoyl-L-Phe, p-Benzoyl-L-Phenylalanine

<400>  51

Tyr His Ser Phe Glu Phe Cys
1               5


<210>  52
<211>  7
<212>  PRT
<213>  Artificial

<220>
<223>  Synthetic peptide with high affinity for Factor VIII and Factor
       VIII-like Proteins

<400>  52

Tyr His Thr Trp Asp Tyr Cys

1               5


<210>  53
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (4)..(4)
<223>  L-Phg, L-Phenylglycine

<400>  53

Ser Trp Glu Xaa Cys
1               5


<210>  54
<211>  7
<212>  PRT
<213>  Artificial

<220>
<223>  Synthetic peptide with high affinity for Factor VIII and Factor
       VIII-like Proteins


<220>
<221>  MOD_RES
<222>  (3)..(3)
<223>  4Abu

<400>  54

Tyr His Xaa Trp Glu Tyr Cys
1               5


<210>  55
<211>  7
<212>  PRT
<213>  Artificial

<220>
<223>  Synthetic peptide with high affinity for Factor VIII and Factor
       VIII-like Proteins


<220>
<221>  MISC_FEATURE
<222>  (6)..(6)
<223>  4-Phenyl-L-Phe, 4-Phenyl-L-Phenylalanine, L-4,4'-Biphenylalanine

<400>  55

Tyr His Ser Trp Glu Phe Cys
1               5


<210>  56
<211>  7
<212>  PRT

<213>  Artificial

<220>
<223>  Synthetic peptide with high affinity for Factor VIII and Factor
      VIII-like Proteins


<220>
<221>  MISC_FEATURE
<222>  (6)..(6)
<223>  L-2-Nal, L-2-Naphthylalanine

<400>  56

Tyr His Ser Trp Glu Xaa Cys
1               5


<210>  57
<211>  5
<212>  PRT
<213>  Artificial

<220>
<223>  Synthetic peptide with high affinity for Factor VIII and Factor
      VIII-like Proteins


<220>
<221>  MISC_FEATURE
<222>  (2)..(2)
<223>  p-Benzoyl-L-Phe, p-Benzoyl-L-Phenylalanine

<220>
<221>  MISC_FEATURE
<222>  (4)..(4)
<223>  L-1-Nal, L-1-Naphthylalanine

<400>  57

Ser Phe Glu Xaa Cys
1               5


<210>  58
<211>  5
<212>  PRT
<213>  Artificial

<220>
<223>  Synthetic peptide with high affinity for Factor VIII and Factor
      VIII-like Proteins


<220>
<221>  MISC_FEATURE
<222>  (2)..(2)
<223>  p-Benzoyl-L-Phe, p-Benzoyl-L-Phenylalanine

<220>
<221>  MISC_FEATURE
<222>  (4)..(4)
<223>  L-2-Nal, L-2-Naphthylalanine

<400>  58

Ser Phe Glu Xaa Cys
1               5

<210>  59
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (2)..(2)
<223>  p-Benzoyl-L-Phe, p-Benzoyl-L-Phenylalanine

<220>
<221>  MISC_FEATURE
<222>  (4)..(4)
<223>  4-Phenyl-L-Phe, 4-Phenyl-L-Phenylalanine, L-4,4'-Biphenylalanine

<400>  59

Ser Phe Glu Phe Cys
1               5


<210>  60
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (2)..(2)
<223>  p-Benzoyl-L-Phe, p-Benzoyl-L-Phenylalanine

<220>
<221>  MISC_FEATURE
<222>  (4)..(4)
<223>  4-Chloro-L-Phe, 4-Chloro-L-Phenylalanine, L-4-Chlorophenylalanine

<400>  60

Ser Phe Glu Phe Cys
1               5


<210>  61
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (2)..(2)
<223>  p-Benzoyl-L-Phe, p-Benzoyl-L-Phenylalanine

<220>

<221>  MISC_FEATURE
<222>  (4)..(4)
<223>  p-Benzoyl-L-Phe, p-Benzoyl-L-Phenylalanine

<400>  61

Ser Phe Glu Phe Cys
1               5


<210>  62
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (2)..(2)
<223>  L-1-Nal, L-1-Naphthylalanine

<220>
<221>  MISC_FEATURE
<222>  (4)..(4)
<223>  4-Phenyl-L-Phe, 4-Phenyl-L-Phenylalanine, L-4,4'-Biphenylalanine

<400>  62

Ser Xaa Glu Phe Cys
1               5


<210>  63
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (2)..(2)
<223>  L-2-Nal, L-2-Naphthylalanine

<220>
<221>  MISC_FEATURE
<222>  (4)..(4)
<223>  4-Phenyl-L-Phe, 4-Phenyl-L-Phenylalanine, L-4,4'-Biphenylalanine

<400>  63

Ser Xaa Glu Phe Cys
1               5


<210>  64
<211>  5
<212>  PRT
<213>  Artificial

<220>
<223>  Synthetic peptide with high affinity for Factor VIII and Factor

VIII-like Proteins

<220>
<221>  MISC_FEATURE
<222>  (2)..(2)
<223>  4-Phenyl-L-Phe, 4-Phenyl-L-Phenylalanine, L-4,4'-Biphenylalanine

<220>
<221>  MISC_FEATURE
<222>  (4)..(4)
<223>  p-Benzoyl-L-Phe, p-Benzoyl-L-Phenylalanine

<400>  64

Ser Phe Glu Phe Cys
1               5

<210>  65
<211>  5
<212>  PRT
<213>  Artificial

<220>
<223>  Synthetic peptide with high affinity for Factor VIII and Factor
       VIII-like Proteins

<220>
<221>  MISC_FEATURE
<222>  (2)..(2)
<223>  3-Benzothienyl-L-Ala, 3-Benzothienyl-L-Alanine,
       L-3-Benzothienylalanine

<220>
<221>  MISC_FEATURE
<222>  (4)..(4)
<223>  p-Benzoyl-L-Phe, p-Benzoyl-L-Phenylalanine

<400>  65

Ser Ala Glu Phe Cys
1               5

<210>  66
<211>  5
<212>  PRT
<213>  Artificial

<220>
<223>  Synthetic peptide with high affinity for Factor VIII and Factor
       VIII-like Proteins

<220>
<221>  MISC_FEATURE
<222>  (2)..(2)
<223>  4-Chloro-L-Phe, 4-Chloro-L-Phenylalanine, L-4-Chlorophenylalanine

<220>
<221>  MISC_FEATURE
<222>  (4)..(4)
<223>  p-Benzoyl-L-Phe, p-Benzoyl-L-Phenylalanine

<400>  66

Ser Phe Glu Phe Cys

1               5


<210>  67
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (2)..(2)
<223>  3,4-Dichloro-L-Phe, 3,4-Dichloro-L-Phenylalanine,
       L-3,4-Dichlorophenylalanine

<220>
<221>  MISC_FEATURE
<222>  (4)..(4)
<223>  p-Benzoyl-L-Phe, p-Benzoyl-L-Phenylalanine

<400>  67

Ser Phe Glu Phe Cys
1               5


<210>  68
<211>  7
<212>  PRT
<213>  Artificial

<220>
<223>  Synthetic peptide with high affinity for Factor VIII and Factor
       VIII-like Proteins

<400>  68

Tyr Tyr Ser Trp Glu Tyr Cys
1               5


<210>  69
<211>  6
<212>  PRT
<213>  Artificial

<220>
<223>  Synthetic peptide with high affinity for Factor VIII and Factor
       VIII-like Proteins


<220>
<221>  MOD_RES
<222>  (1)..(1)
<223>  METHYLATION

<400>  69

Tyr Cys Ser Trp Glu Tyr
1               5


<210>  70
<211>  6
<212>  PRT

<213>  Artificial

<220>
<223>  Synthetic peptide with high affinity for Factor VIII and Factor
       VIII-like Proteins


<220>
<221>  MOD_RES
<222>  (4)..(4)
<223>  METHYLATION

<400>  70

Tyr Cys Ser Trp Glu Tyr
1               5


<210>  71
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (2)..(2)
<223>  p-Benzoyl-L-Phe, p-Benzoyl-L-Phenylalanine

<400>  71

Ser Phe Glu Tyr Cys
1               5


<210>  72
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (2)..(2)
<223>  p-Benzoyl-L-Phe, p-Benzoyl-L-Phenylalanine

<220>
<221>  MISC_FEATURE
<222>  (4)..(4)
<223>  L-Phg, L-Phenylglycine

<400>  72

Ser Phe Glu Xaa Cys
1               5


<210>  73
<211>  5
<212>  PRT
<213>  Artificial

<220>
<223>  Synthetic peptide with high affinity for Factor VIII and Factor
       VIII-like Proteins


<220>
<221>  MISC_FEATURE
<222>  (4)..(4)
<223>  p-Benzoyl-L-Phe, p-Benzoyl-L-Phenylalanine

<400>  73

Ser Trp Glu Phe Cys
1               5


<210>  74
<211>  7
<212>  PRT
<213>  Artificial

<220>
<223>  Synthetic peptide with high affinity for Factor VIII and Factor
       VIII-like Proteins


<220>
<221>  MISC_FEATURE
<222>  (6)..(6)
<223>  L-Phg, L-Phenylglycine

<400>  74

Tyr His Ser Trp Glu Xaa Cys
1               5


<210>  75
<211>  7
<212>  PRT
<213>  Artificial

<220>
<223>  Synthetic peptide with high affinity for Factor VIII and Factor
       VIII-like Proteins


<220>
<221>  MISC_FEATURE
<222>  (4)..(4)
<223>  p-Benzoyl-L-Phe, p-Benzoyl-L-Phenylalanine

<220>
<221>  MISC_FEATURE
<222>  (6)..(6)
<223>  L-Phg, L-Phenylglycine

<400>  75

Tyr His Ser Phe Glu Xaa Cys
1               5


<210>  76
<211>  7
<212>  PRT
<213>  Artificial

<220>
<223>  Synthetic peptide with high affinity for Factor VIII and Factor
      VIII-like Proteins

<400>  76

Tyr His Val Trp Glu Tyr Cys
1               5


<210>  77
<211>  7
<212>  PRT
<213>  Artificial

<220>
<223>  Synthetic peptide with high affinity for Factor VIII and Factor
      VIII-like Proteins


<220>
<221>  MISC_FEATURE
<222>  (4)..(4)
<223>  3,4-Dichloro-L-Phe, 3,4-Dichloro-L-Phenylalanine,
      L-3,4-Dichlorophenylalanine

<400>  77

Tyr His Ser Phe Glu Tyr Cys
1               5


<210>  78
<211>  7
<212>  PRT
<213>  Artificial

<220>
<223>  Synthetic peptide with high affinity for Factor VIII and Factor
      VIII-like Proteins


<220>
<221>  MISC_FEATURE
<222>  (4)..(4)
<223>  3-Benzothienyl-L-Ala, 3-Benzothienyl-L-Alanine,
      L-3-Benzothienylalanine

<400>  78

Tyr His Ser Ala Glu Tyr Cys
1               5


<210>  79
<211>  5
<212>  PRT
<213>  Artificial

<220>
<223>  Synthetic peptide with high affinity for Factor VIII and Factor
      VIII-like Proteins

<400>  79

Tyr Trp Glu Tyr Cys
1               5

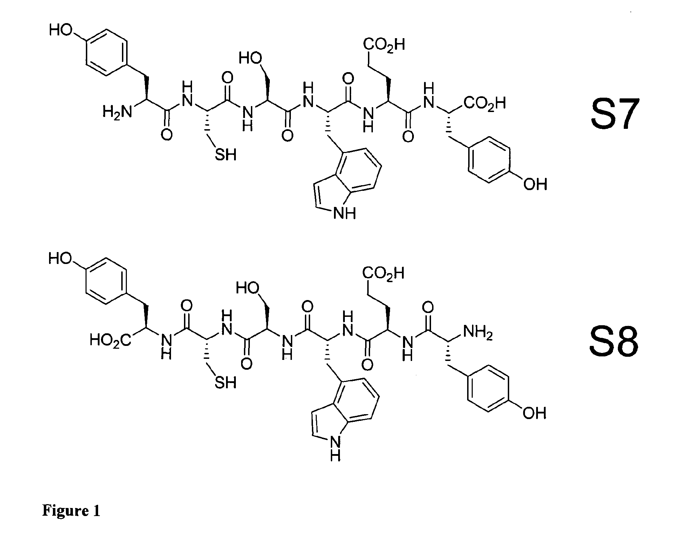

## Claims

1. A compound of the formula I
B-Q-X (I)
wherein
B is selected from a tetrapeptide, pentapeptide, hexapeptide or heptapeptide binding to FVIII or FVIII-like proteins or domains thereof,
Q is absent or an organic spacer molecule, and
X is absent or an organic anchoring molecule,
and pharmaceutically acceptable salts thereof.

2. The compound of Claim 1 wherein
**B** is E¹HN-(Z)ₙ-COE²
wherein
E¹ is H, R¹,-COR¹ or -CO₂R¹,
E² is -OR², -NHR² or -NH-NHR²,
wherein
R¹ is C1-4 alkyl, Ar or CH₂-Ar,
R² is H or R¹,
Ar is an unsubstituted phenyl; or is a substituted phenyl which is one-, two-, or threefold substituted with A, OH, OA, CF₃, OCF₃, CN, NO₂ or Hal, which can be substituted one-, two-, or threefold with an A, OH, OA, CF₃, OCF₃, CN, NO₂ or Hal substituted phenyl, in such a way that an unsubstituted or substituted biphenyl is created; or is Het,
wherein Hal is selected from F, Cl, Br or I,
Het is a saturated or unsaturated mono- or bicyclic heterocyclic residue with 5 to 12 ring members, comprising 1 to 3 N- and/or 1 S- or O- atoms, wherein the heterocyclic residue can be substituted one- or two-fold with CN, Hal, OH , NH₂, COOH, OA, CF₃, A, NO₂, Ar or OCF₃,
A is COOH, NH₂ or alkyl with 1-6 C-atoms, unsubstituted or substituted with COOH or NH₂,
Z is a naturally occurring or non-proteinogenic amino acid residue or derivative thereof,
n is an integer between 4 and 7,
wherein
Z is linked by a bond selected from the group of an acid-amide bond -CO-NR³-or -NR³-CO-, a reduced peptide bond -CH₂-NR³- or -NR³-CH₂-, a bond -CO-CHR³-, -CHR³-CO-, -CR³=CH- or a -CH=CR³- bond,
wherein R³ is selected from H, C₁₋₄alkyl, phenyl or benzyl or, in case of peptoid-amino acids, the amino acid side chain,
and pharmaceutically acceptable salts thereof.

3. The compound of Claim 1 or 2, wherein Q is an organic spacer molecule selected from
[-NH-(CH₂)ₓCO]_{w},
[-NH-(CH₂CH₂-O-)yCH₂-CO]_{w},
[CO-(CH₂)₂CO-],
[NH-(CH₂)_{z}-NH-],
[CO-CH₂-(OCH₂CH₂)_{y}-O-CH₂-CO-],
[NH-CH₂CH₂-(OCH₂CH₂)_{y}-NH-],
and combinations thereof
wherein
w is an integer from between 1 to 8,
x is an integer from between 1 to 5,
y is an integer from between 1 to 6, and
z is an integer from between 1 to 6.
and pharmaceutically acceptable salts thereof.

4. The compound of Claim 1, 2 or 3, wherein X is an organic anchoring molecule selected from the group consisting of
a naturally occurring or non-proteinogenic amino acid,
-A¹-(CH₂)ₚ-A²,
-A¹-CH₂-(OCH₂CH₂)_{y}-O-CH₂-A²,
-A¹-CH₂CH₂-(OCH₂CH₂)_{y}-A²,
=CR²-(CH₂)ₚ-A²,
-A¹-CH(NHR⁴)-(CH₂)_{q}-A²,
=CR²-CH(NHR⁴)-(CH₂)_{q}-A²,
-A¹-CH(COR⁵)-(CH₂)_{q}-A² or
=CR²-CH(COR⁵)-(CH₂)_{q}-A²,
wherein
A¹ is NR², CO, CHR², O, or S,
A² is SH, N₃, NH-NH₂, O-NH₂, NH₂, Hal¹, C=CH, CR⁶O, or carboxyl,
R² is as defined above in Claim 2,
R⁴ is H, R⁶, -COR⁶, or -COOR⁶,
R⁵ is -OR⁶ or -NHR⁴,
R⁶ is H, C₁₋₄alkyl; or is unsubstituted phenyl or with A, OH, OA, CF₃, OCF₃, CN, NO₂ or Hal one-, two-, or threefold substituted phenyl; or benzyl,
p is an integer from between 1 to 20,
q is an integer from between 1 to 20,
y is an integer from between 1 to 6,
z is an integer from between 1 to 6,
Hal is an integer from between as defined above in Claim 2, and
Hal¹ is Cl, Br or I.

5. The compound according to any one of Claims 1 to 4,
wherein one of Z from (Z)ₙ with n between 4 to 7 is selected from alanine, valine, serine, threonine or α-aminobutyric acid, and/or one of Z from (Z)ₙ with n between 4 to 7 is selected from cysteine, *homo*-cysteine or D-cysteine, and/or one of Z from (Z)ₙ with n between 4 to 7 is selected from glutamic or aspartic acid.

6. The compound according to any one of Claims 1 to 5,
wherein one of Z from (Z)ₙ with n between 1 to 6 is selected from phenylalanine, tyrosine, O-methylated tyrosine, 1-naphthylalanine, 2-naphthylalanine, tryptophane, p-benzoylphenylalanine or an -CH₂-Ar, wherein Ar is defined as in Claim 2.

7. The compound according to any one of Claims 1 to 6, wherein E¹ is H or acetyl, and/or E² is -OH or NH₂.

8. The compound according to any one of Claims 1 to 7, wherein
Q is [-NH-(CH₂)ₓ-CO]_{w}
and x and w are as defined above in Claim 3.

9. The compound according to any one of Claims 1 to 8, wherein x is 1 or 5, and w is 0 or 1.

10. The compound according to any one of Claims 1 to 9, wherein
X is -A¹-CH(COR⁵)-(CH₂)_{q}-A², wherein A¹ is NH, R⁵ is OH or NH₂, A² is SH, and q is as defined above in Claim 4, or
X is -A¹-CH(NHR⁴)-(CH₂)_{q}-A², wherein A¹ is CO, R⁴ is H, A² is SH, and q is as defined above in Claim 4.

11. The compound according to any one of Claims 1 to 10, wherein q is 1.

12. The compound according to any one of Claims 1 to 11, wherein the amino acid residues can be independently selected from an L- or D-α-amino carbonic acid, an β-amino carbonic acid, an aza-amino carbonic acid, and a peptoid-amino carbonic acid.

13. The compound according to any one of Claims 1 to 12, comprising one or more modified peptide bonds according to Claim 1.

14. The compound according to any one of Claims 1 to 13, in the form of a retropeptide, wherein the amino acid residues are preferably chosen from D-α-amino acid residues ("retro-inverso-peptide").

15. A pharmaceutical composition, comprising a compound according to any of claims 1 to 14, wherein said compound is selected from the group
| | |
|---|---|
| S1 | **Y-C-S-W-E-Y-NH₂** |
| S2 | **Ac-Y-C-S-W-E-Y-NH₂** |
| S3 | **Y-C-T-W-D-Y-NH₂** |
| S4 | **Ac-Y-C-T-W-D-Y-NH₂** |
| S5 | **Y-*homo*C-S-W-E-Y** |
| S6 | **Y-c-S-W-E-Y** |
| S7 | **Y-C-S-W-E-Y** |
| S8 | **y-e-w-s-c-y** |
| S9 | **Ac-Y-C-S-W-E-Y** |
| S10 | **Y-C-T-W-E-Y** |
| S11 | **Y-C-S-W-D-Y** |
| S12 | **Y-C-T-W-D-Y** |
| S13 | **y-d-w-t-c-y** |
| S14 | **Ac-Y-C-T-W-D-Y** |
| S15 | **Y-C-S-Bpa-E-Y** |
| S16 | **Y-C-A-W-D-Y** |
| S17 | **y-d-w-a-c-y** |
| S18 | **Y-C-V-W-D-Y** |
| S19 | **y-d-w-v-c-y** |
| S20 | **C-S-W-E-Y** |
| S21 | **(*N*-Me)S-W-E-Y-***C* |
| S22 | **Y-F-(*N*-Me)S-W-E-Y-***C* |
| S23 | **Y-F-S-W-(*N*-Me)E-Y-***C* |
| S24 | **Y-C-(*N*-Me)S-W-E-Y** |
| S25 | **Y-C-S-W-(*N*-Me)E-Y** |
| S26 | **Y-C-S-W-E-(*N*-Me)Y** |
| S27 | **Y-f-(*N*-Me)S-W-E-Y-***C* |
| S28 | **Y-f-S-W-(*N*-Me)E-Y-***C* |
| S29 | **H-S-W-E-Y-*C*** |
| S30 | **Ac-S-W-E-Y-*C*** |
| S31 | **Ac-H-S-W-E-Y-*C*** |
| S32 | **Ac-Y-H-S-W-E-Y-*C*** |
| S33 | **Y-F-Abu-W-E-F-*C*** |
| S34 | **Y-f-S-W-E-Y-*C*** |
| S35 | **c-y-e-w-s** |
| S36 | **Y-F-Abu-W-E-Y-***C* |
| S37 | **Y-H-S-Bpa-E-Y-***C* |
| S38 | **Y-H-S-W-E-Bpa-***C* |
| S39 | **Y-H-Abu-W-D-Y-***C* |
| S40 | **Y-S-W-E-Y-***C* |
| S42 | **Y-H-S-(2-Nal)-E-Y-***C* |
| S43 | **S-W-D-Y-***C* |
| S44 | **E-S-W-E-Y-***C* |
| S45 | **Ac-E-S-W-E-Y-***C* |
| S46 | **T-W-E-Y-***C* |
| S47 | **Y-H-T-W-E-Y-***C* |
| S48 | **Y-H-S-W-D-Y-***C* |
| S49 | **Y-F-Abu-W-E-Y-***C* |
| S50 | **Abu-W-E-Y-***C* |
| S51 | **Y-H-S-Bpa-E-Bpa-***C* |
| S52 | **Y-H-T-W-D-Y-***C* |
| S53 | **S-W-E-Phg-C** |
| S54 | **Y-H-Abu-W-E-Y-***C* |
| S55 | **Y-H-S-W-E-Bip(4,4')-***C* |
| S56 | **Y-H-S-W-E-(2-Nal)-***C* |
| S57 | **S-Bpa-E-(1-Nal)-***C* |
| S58 | **S-Bpa-E-(2-Nal)-***C* |
| S59 | **S-Bpa-E-Bip(4,4')-***C* |
| S60 | **S-Bpa-E-Phe(4-Cl)-***C* |
| S61 | **S-Bpa-E-Bpa-C** |
| S62 | **S-(1-Nal)-E-Bip(4,4')-***C* |
| S63 | **S-(2-Nal)-E-Bip(4,4')-C** |
| S64 | **S-Bip(4,4')-E-Bpa-C** |
| S65 | **S-Bta-E-Bpa-C** |
| S66 | **S-Phe(4-Cl)-E-Bpa-***C* |
| S67 | **S-Phe(3,4-Cl)-E-Bpa-C** |
| S68 | **Y-Y-S-W-E-Y-***C* |
| S69 | **(*N*-Me)Y-C-S-W-E-Y** |
| S70 | **Y-C-S-(*N*-Me)W-E-Y** |
| S71 | **S-Bpa-E-Y-***C* |
| S72 | **S-Bpa-E-Phg-C** |
| S73 | **S-W-E-Bpa-***C* |
| S74 | **Y-H-S-W-E-Phg-***C* |
| S75 | **Y-H-S-Bpa-E-Phg-***C* |
| S76 | **Y-H-V-W-E-Y-***C* |
| S77 | **Y-H-S-Phe(3,4-Cl)-E-Y-***C* |
| S78 | **Y-H-S-Bta-E-Y-***C* |
| S79 | **Y-W-E-Y-***C* |
and a suitable carrier.

16. The compound according to any one of Claims 1 to 14 or the pharmaceutical composition according to claim 15 for the treatment of diseases.

17. A solid carrier matrix comprising a compound according to any one Claims 1 to 14 that is attached to said matrix.

18. The solid carrier matrix according to Claim 17, wherein said attachment of said compound is through an organic spacer molecule or organic anchoring molecule.

19. A diagnostic device or kit, comprising the compound according to any one of Claims 1 to 14, the pharmaceutical composition according to claim 15, the support matrix according to Claim 17 or 18, and, optionally, auxiliary diagnostic means, such as a detectable label.

20. A method of detecting, identifying, diagnosing, isolating, purifying, stabilizing and/or enhancing the activity of a FVIII or FVIII-like protein or domains thereof, comprising contacting a sample comprising a FVIII or FVIII-like protein or their domains with the solid carrier matrix according to Claim 17 or 18, under conditions suitable for attachment between FVIII or FVIII-like protein or domains thereof and said solid carrier matrix, and detecting, identifying, diagnosing, isolating, purifying, stabilizing and/or enhancing the activity of said FVIII or FVIII-like protein or their domains on or from said matrix.

21. The method of Claim 20, wherein said compound is selected from the group of S1, S2, S3, S4, S5, S6, S7, S8, S9, S10, S11, S12, S13, S14, S15, S16, S16, S17, S18, S19, S20, S21, S22, S23, S24, S25, S26, S27, S28, S29, S30, S31, S32, S33, S34, S35, S36, S37, S38, S39, S40, S42, S43, S44, S45, S46, S47, S48, S49, S50, S51, S52, S53, S54, S55, S56, S57, S58, S59, S60, S61, S62, S63, S64, S65, S66, S67, S68, S69, S70, S71, S72, S73, S74, S75, S76, S77, S78, and S79 as defined in claim 24.

22. The method according to Claim 20 or 21, wherein said sample is a cell culture supernatant or body fluid, such as serum or whole blood.

23. A method for producing a FVIII or FVIII-like protein or domains thereof containing a pharmaceutical composition or medicament, comprising a method for purifying a FVIII or FVIII-liKe protein or domains thereof according to any of Claims 21 to 23, and formulating said purified FVIII or FVIII-like protein or their domains.

24. A method for producing and/or stabilizing a FVIII or FVIII-like protein or their domains containing a pharmaceutical composition or medicament, comprising a method for purifying and/or stabilizing a FVIII or FVIII-like protein or domains thereof according to any of Claims 21 to 23, and formulating said purified and/or stabilized FVIII or FVIII-like protein or their domains.

25. A method for enhancing the activity of a FVIII or FVIII-like protein or domains thereof containing a pharmaceutical composition or medicament, comprising a method for enhancing the activity of a FVIII or FVIII-like protein or domains thereof according to any of Claims 21 to 23, and formulating said activity enhanced FVIII or FVIII-like protein or their domains.

26. Use of the compound according to any of Claims 1 to 14 for labeling, detecting, diagnosing, monitoring, identifying, isolating, purifying, stabilizing and/or enhancing the activity of a FVIII or FVIII-like protein or domains thereof.
